(19) Europäisches Patentamt
European Patent Office
Office européen des brevets

(11) EP 0 851 287 B1

(12) EUROPEAN PATENT SPECIFICATION

(45) Date of publication and mention of the grant of the patent:
**27.03.2002 Bulletin 2002/13**

(51) Int Cl.7: **G03C 5/44**, G03C 7/42, C07C 229/24, C07C 229/76

(21) Application number: **97122858.0**

(22) Date of filing: **23.12.1997**

(54) **Aminopolycarboxylic acid-series chelating agent, heavy metal compound thereof, photographic additive, and processing method**

Aminopolycarboxylat-Chelatbildner, Schwermetall-Verbindung davon, photographischer Zusatz und Verarbeitungsverfahren

Agent chélant aminopolycarboxylé, son composé métallique lourd, additif photographique et procédé de traitement

(84) Designated Contracting States:
**AT BE CH DE DK ES FI FR GB GR IE IT LI LU MC NL PT SE**

(30) Priority: **27.12.1996 JP 35675096**

(43) Date of publication of application:
**01.07.1998 Bulletin 1998/27**

(73) Proprietor: **Fuji Photo Film Co., Ltd.**
**Kanagawa-ken (JP)**

(72) Inventors:
- **Inaba, Tadashi**
  **Minami-ashigara-shi, Kanagawa-ken (JP)**
- **Hirano, Shigeo**
  **Minami-ashigara-shi, Kanagawa-ken (JP)**
- **Morimoto, Kiyoshi**
  **Minami-ashigara-shi, Kanagawa-ken (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 175 180** **US-A- 5 585 226**

- **M. MORIGUCHI, Y. UMEDA, K. MIYAZAKI, T. NAKAMURA, K. OGAWA, F. KOJIMA, H. IINUMA AND T. AOYAGI: "SYNTHESIS OF HISTARGIN AND RELATED COMPOUNDS AND THEIR INHIBITION OF ENZYMES" JOURNAL OF ANTIBIOTICS, vol. XLI, no. 12, 1988, pages 1823-1827, XP002099301**
- **H. BRÜCKNER AND C. GAH: "High-performance liquid chromatographic separation of DL-amino acids derivatized with chiral variants of Sanger's reagent" JOURNAL OF CHROMATOGRAPHY, vol. 555, no. 1+2, 1991, pages 81-95, XP002099302**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 851 287 B1

## Description

### FIELD OF THE INVENTION

The present invention relates to aminopolycarboxylic acid-series chelating agents and their heavy metal chelate compounds, and to a method for processing a silver halide photographic light-sensitive material, in which method use is made of them. More specifically, the present invention relates to chelating agents for sequestering metal ions harmful to photographic processing.

### BACKGROUND OF THE INVENTION

Light-sensitive materials are processed with a processing solution having a bleaching capacity after they are exposed to light and subjected to color-development. As bleaching agents contained in the processing solution having a bleaching capacity, ferric complex salts are widely known, and particularly out of them, the ferric complex salt of ethylenediaminetetraacetic acid (EDTA) has long been used, and the ferric complex salt of 1,3-propanediaminetetraacetic acid (1,3-PDTA), having a more powerful bleaching capacity, has become used widely in recent years. Although, compared to the ferric complex salt of EDTA, the ferric complex salt of 1,3-PDTA is excellent in rapid processibility, bleach fogging is liable to occur because of its strong oxidation power, and the image storage characteristics after the processing is easily deteriorated (resulting in an increase in magenta stain). Further, since the oxidation-reduction potential is high, a system containing thiosulfuric acid has some problems because, for example, decomposition of thiosulfuric acid is facilitated with time, to deposit sulfur. Further, in view of the growing awareness of preservation of the earth's environment in recent years, in the photographic industry, wherein the development of processing agents low in environmental pollution load is required, the development of bleaching agents in place of the ferric complex salt of EDTA and the ferric complex salt of 1,3-PDTA, which are difficult to biodegrade, is under way. Further, although these metal complex salts are used, besides in bleaching solution compositions, in processing compositions for post-processing, for example, for intensification, reduction, or toning, the problem of biodegradability remains unsolved.

In recent years, as compounds for solving these problems, those described in JP-A-5-72695 ("JP-A" means unexamined published Japanese patent application), JP-A-5-303186, and US-A-5 585 226 have been developed. However, it has been found that use of these compounds brings about new problems. For example, when the ferric complex of ethylenediaminedisuccinic acid (EDDS), i.e. the compound described in JP-A-5-72695 and JP-A-5-303186, or the ferric complex of ethylenediamine-N-carboxymethyl-N'-monosuccinic acid, described in US-A-5 585 226, are used in a bleach-fix solution, there occur such problems as insufficiency of color formation (restoration) and fading (discoloration) due to the bleach-fix solution. To solve the problems, further earnest development efforts are required. Furthermore, ethylenediaminedisuccinic acid (EDDS) and ethylenediamine-N-carboxymethyl-N'-monosuccinic acid are compounds that are not very easily biodegraded, and to decompose them, relatively powerful biodegradation conditions are required. Thus, in view of preservation of the environment, compounds that can be more easily biodegraded have been desired.

On the other hand, as the above photographic processing step is carried out in various places, ranging from a large-scale photofinishing laboratory, provided with a large-sized automatic processor, to a photo processing shop having a small-sized automatic processor, called a mini-lab, on a counter, which has appeared in recent years, the processing performance has been lowered sometimes.

One of the major causes thereof is mixing of metal ions in processing solutions. The influence of the mixed ions varies depending on the ions and the processing solution, and they cause conspicuous lowering of photographic properties through clogging of filters in a circulating system of an automatic processor, processing stains of films, and decomposition, for example, of a color-developing agent, a black-developing agent, such as hydroquinone and Monole (trade name), and a preservative, such as hydroxylamines and sulfites.

To solve the foregoing problems, chelating agents for sequestering metal ions has been used. Examples include aminopolycarboxylic acids (e.g. ethylenediaminetetraacetic acid and diethylenetriaminepentaacetic acid), described in JP-B-48-30496 ("JP-B" means examined Japanese patent publication) and JP-B-44-30232; organic phosphonic acids, described in JP-A-56-97347, JP-B-56-39359, and DE-C-2 227 639; phosphonocarboxylic acids, described, for example, in JP-A-52-102726, JP-A-53-42730, JP-A-54-121127, JP-A-55-126241, and JP-A-55-65956; and compounds described, for example, in JP-A-58-195845, JP-A-58-203440, and JP-B-53-40900. Some of these compounds has been practically used, but their performance is not satisfactory.

Further, a compound having a skeleton similar to that of the compound of the present invention is ethylenediamine-N,N'-disuccinic acid, described on pages 309 to 311 of Chelate Chemistry (Kireto Kagaku) (5), written by Kagehira Ueno (Nankodo).

The sequestering capacity of this compound changes greatly depending on the pH, and when the conditions

of the processing solution change, the level of performance becomes unsatisfactory in many cases. In particular, because the sequestering capacity of the compound for iron ions is inherently low in an alkaline developing bath, it is required to add an excess amount of the compound, which is not preferable on photographic performances. Accordingly, development of a novel excellent chelating agent whose sequestering effect is much better and that is not affected by a change in conditions, has been particularly desired.

## SUMMARY OF THE INVENTION

Therefore, a first object of the present invention is to provide a chelating agent for various metals that is excellent in biodegradability and metal sequestering capacity.
A second object of the present invention is to provide a heavy metal chelate compound excellent in biodegradability.
A third object of the present invention is to provide a photographic additive that does not bring about precipitation or the formation of sludge, even when metal ions are mixed therein.
A fourth object of the present invention is to provide a chelating agent whose metal sequestering capacity is changed little by a change in the pH.
A fifth object of the present invention is to provide a additive that does not bring about such problems as insufficiency of color formation and fading caused by a bleach-fix solution.
Other and further objects, features, and advantages of the invention will appear more fully from the following description.

## DETAILED DESCRIPTION OF THE INVENTION

The inventors of the present invention, having intensively studied the above problems, have found that the above objects can be achieved by providing the below-described aminopolycarboxylic acid-series chelating agent, its heavy metal chelate compound, and a method for processing a silver halide photographic light-sensitive material, in which method they are used. Thus, the present invention provides:

(1) A compound represented by the following formula (I):

formula (I)

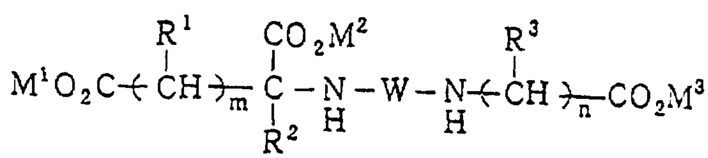

wherein $R^1$ and $R^2$ each represent a hydrogen atom, an alkyl group, an aryl group, a heterocyclic group, a carboxyl group, an amino group, an alkoxy group, a sulfo group, a nitro group, a phosphono group, an acylamino group, a sulfonylamino group, an aryloxy group, a sulfamoyl group, a carbamoyl group, an alkylthio group, an arylthio group, an acyl group, a hydroxamic acid group, or a hydroxyl group; $R^3$ represents a hydrogen atom, an alkyl group substituted with at least one selected from a group consisting of an alkoxy group and an aryl group, an unsubstituted alkyl group, an aryl group, or a heterocyclic group with the proviso that $R^3$ does not represent an isopropyl group; W represents a divalent linking group having a carbon atom(s); m and n are each an integer of 1 to 3, with the proviso that when n = 1, $R^3$ does not represent a hydrogen atom, when n = 2 or 3, at least one of $nR^3$'s represents an alkyl group, an aryl group or a heterocyclic group, and when m and n are each 2 or more, $mR^1$'s are the same or different and $nR^3$'s are the same or different, and $M^1$, $M^2$, and $M^3$ each represent a hydrogen atom or a cation;

(2) A compound of formula (I) with the provise that, when n = 2 or 3, one of $nR^3$'s represents an alkyl group, an aryl group or a heterocyclic group and the other $R^3$'s all represent hydrogen atoms and when m is 2 or more, $mR^1$'s are the same or different, and $M^1$, $M^2$, and $M^3$ each represent a hydrogen atom or a cation;

(3) A heavy metal chelate compound of the compound represented by formula (I) stated in the above (1);

(4) Use of a compound represented by formula (I) stated in the above (1) as a chelating agent;

(5) A photographic additive, comprising a compound represented by formula (I) stated in the above (1) or its heavy metal chelate compound; and

(6) A method for processing a silver halide photographic light-sensitive material, comprising processing a silver halide photographic light-sensitive material, which has been exposed imagewise, in the presence of at least one compound out of the compounds represented by formula (I) or their heavy metal chelate compounds.

Herein , in the present invention, the group on the compound includes both a group having a substituent thereon and a group having no substituent (i.e. an unsubstituted group), unless otherwise specified.

The feature of the structure of formula (I) is that it has at least two secondary amine structures and at least three carboxyl groups. It is also characterized in that there is a carboxyalkyl group, which is substituted by a heterocyclic group, an aryl group or an alkyl group, on one N, and there is a group having two carboxyl groups with at least two carbon atoms between them (e.g. a succinic acid group) on the other N'.

Hitherto, as compounds having a similar skeleton, there are compounds described in JP-A-5-72695 and JP-A-5-303186 (e.g. ethylenediaminedisuccinic acid). However, these compounds, as bleaching agents and metal sequestering agents, cannot solve all of the previously described problems. Further, compounds described in US-A-5 585 226 (e.g. ethylenediamine-N-carboxymethyl-N'-monosuccinic acid) also cannot solve all of the previously described problems. It is new and unexpected that the previously described problems can be solved by substituting the carboxyalkyl group substituted on one of the N's by an alkyl group, an aryl group, or a heterocyclic group, as in the compound of the present invention. Particularly with respect to biodegradability, it is generally found that compounds having a structure with a branch, such as an alkyl group, are poor in biodegradability in comparison with compounds having no branch, and the inventors of the present invention have, until now, also obtained this result. However, contrary to the expectation of the biodegradability, the compounds now found, which are branched (substituted), can be biodegraded very easily. This result is contrary to the general sense and is quite unexpected.

Hereinbelow, the compound represented by formula (I) will be described first in detail.

Examples of the alkyl group represented by $R^1$ and $R^2$ include a straight-chain, branched, or cyclic alkyl group, and preferably the alkyl group is a straight-chain or branched alkyl group having 1 to 10 carbon atoms, more preferably a straight-chain or branched alkyl group having 1 to 5 carbon atoms, and most preferably a straight-chain alkyl group having 1 to 5 carbon atoms. Examples are methyl, ethyl, n-propyl, and n-butyl.

The alkyl group may be substituted. Examples of the substituent include an aryl group (e.g. phenyl and naphthyl, with preference given to phenyl; and the aryl group may be substituted), a heterocyclic group (e.g. 2-pyridyl, 3-pyridyl, 4-pyridyl, 4-imidazolyl, 2-imidazolyl, 3-pyrazolyl, 4-pyrazolyl, 2-thiazolyl, 4-thiazolyl, 2-furyl, 3-furyl, 2-thienyl, 3-thienyl, 2-pyrrolyl, 3-pyrrolyl, 2-oxazolyl, 4-oxazolyl, 2-pyrazinyl, 2-pyrrolidinyl, 2-indolyl, 3-indazonyl, 2-quinolyl, 8-quinolyl, piperidino, morpholino, 2-piperidyl, 2-morpholinyl, 3-indazolyl, 2-purinyl, 6-purinyl, 2-(1,3,4-thiazolyl), 2-(1,3,4-oxadiazolyl), 1-phthalazyl, 2-quinoxalinyl, 5-quinoxalinyl, 2-quinazolinyl, 4-quinazolinyl, 8-quinazolinyl, 3-cinnolinyl, 8-cinnolinyl, 2-(1,10-phenanthrolinyl), and 5-tetrazolyl, with preference given to 2-pyridyl, 4-imidazolyl, 2-imidazolyl, 3-pyrazolyl, 2-thiazolyl, 2-pyrrolyl, 2-oxazolyl, 4-oxazolyl, 2-pyrazinyl, 2-pyrrolidinyl, 2-indolyl, 3-indazonyl, 2-quinolyl, 8-quinolyl, piperidino, 2-piperidyl, 2-indolyl, 3-indazolyl, 2-(1,3,4-thiazolyl), and 2-(1,3,4-oxadiazolyl), and more preferably to 4-imidazolyl, 2-imidazolyl, and 2-pyridyl; and the heterocyclic group may be substituted), a carboxyl group, an alkoxy group (which alkoxy group has preferably 1 to 8 carbon atoms, more preferably 1 to 6 carbon atoms, and particularly preferably 1 to 4 carbon atoms, such as methoxy and ethoxy), a sulfo group, a nitro group, a phosphono group, a hydroxamic acid group, a hydroxyl group, an aryloxy group (preferably having 6 to 12 carbon atoms, more preferably 6 to 10 carbon atoms, and particularly preferably 6 to 8 carbon atoms, such as phenyloxy), a sulfamoyl group (preferably having 0 to 10 carbon atoms, more preferably 0 to 6 carbon atoms, and particularly preferably 0 to 4 carbon atoms, such as sulfamoyl and methylsulfamoyl), a carbamoyl group (preferably having 1 to 10 carbon atoms, more preferably 1 to 6 carbon atoms, and particularly preferably 1 to 4 carbon atoms, such as carbamoyl and methylcarbamoyl), an alkylthio group (which alkylthio group has preferably 1 to 8 carbon atoms, more preferably 1 to 6 carbon atoms, and particularly preferably 1 to 4 carbon atoms, such as methylthio and ethylthio), an arylthio group (preferably having 6 to 20 carbon atoms, more preferably 6 to 10 carbon atoms, and particularly preferably 6 to 8 carbon atoms, such as phenylthio), a mercapto group, an acyl group (preferably having 2 to 10 carbon atoms, more preferably 2 to 6 carbon atoms, and particularly preferably 2 to 4 carbon atoms, such as acetyl and benzoyl), an acylamino group (preferably having 1 to 10 carbon atoms, more preferably 2 to 6 carbon atoms, and particularly preferably 2 to 4 carbon atoms, such as acetylamino), a sulfonylamino group (preferably having 1 to 10 carbon atoms, more preferably 1 to 6 carbon atoms, and particularly preferably 1 to 4 carbon atoms, such as methanesulfonylamino), and an amino group (preferably having 0 to 20 carbon atoms, more preferably 0 to 10 carbon atoms, and particularly preferably 0 to 6 carbon atoms, such as amino, methylamino, ethylamino, dimethylamino, and diethylamino). Preferably the substituent is an aryl group, a heterocyclic group, or an amino group, and more preferably an aryl group or a heterocyclic group.

The aryl group represented by $R^1$ and $R^2$ is a monocyclic or dicyclic aryl group, such as a phenyl group and a naphthyl group, with preference given to a phenyl group. The aryl group may be substituted, and examples of the substituent include an alkyl group, as well as the substituent that may be possessed by the alkyl group represented by $R^1$ and $R^2$.

As the heterocyclic group represented by $R^1$ and $R^2$, for example, the heterocyclic group that may be possessed by the alkyl group represented by $R^1$ and $R^2$ can be suitably used. Preferable heterocyclic groups are the same as those that may be preferably possessed by the alkyl group represented by $R^1$ and $R^2$. The heterocyclic group may be substituted, and examples of the substituent include an alkyl group, as well as the substituent that may be possessed by the alkyl group represented by $R^1$ and $R^2$.

Examples of the amino group, the alkoxy group, the acylamino group, the sulfonylamino group, the aryloxy group, a sulfamoyl group, the carbamoyl group, the alkylthio group, the arylthio group, and the acyl group, represented by $R^1$ and $R^2$, include the substituents that may be possessed by the above alkyl group represented by $R^1$ and $R^2$, and preferable ones are the same as those that may be preferably possessed by the alkyl group represented by $R^1$ and $R^2$.

Preferably $R^1$ and $R^2$ each represent a hydrogen atom, an alkyl group, an aryl group, a heterocyclic group, or a hydroxyl group; more preferably a hydrogen atom or a hydroxyl group, and particularly preferably a hydrogen atom.

Examples of the alkyl group represented by $R^3$ include a straight-chain, branched or cyclic alkyl group, and preferably the alkyl group is a straight or branched alkyl group having 1 to 10 carbon atoms, more preferably a straight-chain or branched alkyl group having 1 to 5 carbon atoms, and most preferably a straight-chain alkyl group having 1 to 5 carbon atoms. Examples are methyl, ethyl, n-propyl, and n-butyl. Isopropyl is excluded.

The alkyl group may be substituted. Examples of the substituent include an aryl group (e.g. phenyl and naphthyl with preference given to phenyl; and the aryl group may be substituted), or an alkoxy group (which alkoxy group has preferably 1 to 8 carbon atoms, more preferably 1 to 6 carbon atoms, and particularly preferably 1 to 4 carbon atoms, such as methoxy and ethoxy). Preferably the substituent for an alkyl group is an aryl group.

The aryl group represented by $R^3$ is a monocyclic or dicyclic aryl group, such as a phenyl group and a naphthyl group, with preference given to a phenyl group. The aryl group may be substituted, and examples of the substituent include an alkyl group, as well as the substituent that may be possessed by the alkyl group represented by $R^1$ and $R^2$.

As the heterocyclic group represented by $R^3$, for example, the heterocyclic group that may be possessed by the alkyl group represented by $R^1$ and $R^2$ can be suitably used. Preferable heterocyclic groups are the same as those that may be preferably possessed by the alkyl group represented by $R^1$ and $R^2$. The heterocyclic group may be substituted, and examples of the substituent include an alkyl group, as well as the substituent that may be possessed by the alkyl group represented by $R^1$ and $R^2$.

Preferably, $R^3$ is an unsubstituted alkyl group or an aryl group, and more preferably an unsubstituted alkyl group.

The divalent linking group represented by W can preferably be represented by the following formula (W):

formula (W)

$$-(W^1-D)_s-(W^2)_t-$$

wherein $W^1$ and $W^2$, which are the same or different, each represent a straight-chain or branched alkylene group having 2 to 8 carbon atoms (e.g. ethylene, and propylene), a cycloalkylene group having 5 to 10 carbon atoms (e.g. 1,2-cyclohexyl), an arylene group having 6 to 10 carbon atoms (e.g. o-phenylene), an aralkylene group having 7 to 10 carbon atoms (e.g. o-xylenyl), or a carbonyl group. D represents -O-, -S-, or $-N(R_W)-$, or a divalent nitrogen-containing heterocyclic group.

$R_W$ represents a hydrogen atom or an alkyl group having 1 to 8 carbon atoms (e.g. methyl), or an aryl group having 6 to 10 carbon atoms (e.g. phenyl), each of which may be substituted by $-COOM^a$, $-PO_3M^bM^c$, -OH, or $-SO_3M^d$. $M^a$, Mb, $M^c$, and $M^d$ each represent a hydrogen atom or a cation.

As the divalent nitrogen-containing heterocyclic group, a 5- or 6-membered heterocyclic group whose hetero atom is a nitrogen atom, is preferable, and one that is bonded to $W^1$ and $W^2$ at the carbon atoms that are adjacent to each other, such as an imidazolyl group, is more preferable.

$W^1$ and $W^2$ preferably each represent an alkylene group having 2 to 4 carbon atoms. s is an integer of 0 to 3. When s is 2 or 3, $W^1$-D's are the same or different. s is preferably 0 to 2, more preferably 0 or 1, and particularly preferably 0. t is an integer of 1 to 3. When t is 2 or 3, $W^2$'s are the same or different. t is preferably 1 or 2.

The linking group represented by W may be substituted, and examples of the substituent include an alkyl group (for example, those mentioned in the above alkyl group represented by $R^1$ and $R^2$, and preferable ones are the same as those mentioned in the above), an aryl group (including a phenyl group and a naphthyl group, with preference given to a phenyl group; and the aryl group may be substituted), a heterocyclic group (including, for example, the heterocyclic groups that may be possessed by the alkyl group represented by $R^1$ and $R^2$, and preferable ones are the same as those mentioned in. the above; and the heterocyclic group may be further substituted), an alkoxy group (preferably having 1 to 8 carbon atoms, more preferably 1 to 6 carbon atoms, and particularly preferably 1 to 4 carbon

atoms, such as methoxy and ethoxy), and a hydroxyl group, with preference given to an alkyl group and a hydroxyl group, and more preferably to an alkyl group. Preferably the linking group represented by W is unsubstituted.

Specific examples of W include, for example, the following:

$-(CH_2)_2-$ , $-(CH_2)_3-$ , $-(CH_2)_4-$ ,

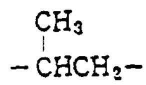 , 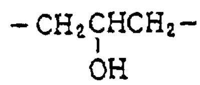 ,

$-CH_2CH_2CH_2OCH_2CH_2-$ ,

$-CH_2CH_2OCH_2CH_2OCH_2CH_2-$ ,

$-CH_2CH_2SCH_2CH_2-$ ,

$-CH_2CH_2SCH_2CH_2SCH_2CH_2-$ ,

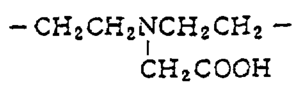 ,

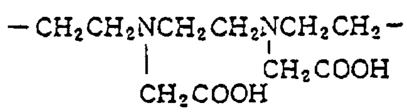 ,

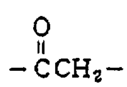 , 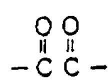 ,

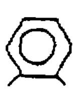 , 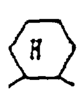 , 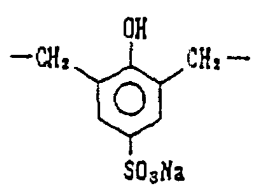 ,

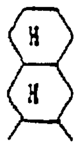 , 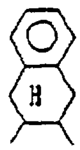 , 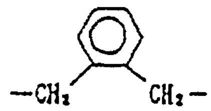 ,

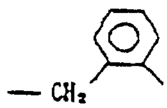 , 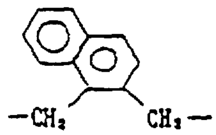 , 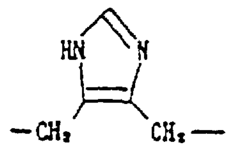 ,

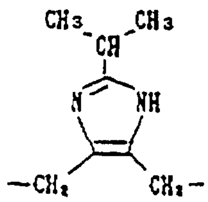 , 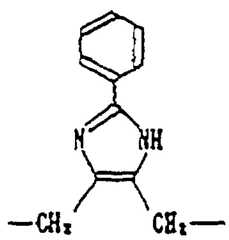 , 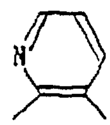 , $-CH_2-CH=CH-CH_2-$ .

m is an integer of 1 to 3, preferably 1 or 2, and more preferably 1.

n is an integer of 1 to 3, preferably 1 or 2, and more preferably 1.

The cation represented by $M^1$, $M^2$, and $M^3$ may be an organic cation or an inorganic cation. If there are two or more cations in one molecule, the cations may be different. Examples of the cation include an ammonium (e.g. an ammonium and a tetraethylammonium), an alkali metal (e.g. a lithium, a sodium, and a potassium), an alkaline earth metal (e.g. a calcium, a magnesium, and a barium), and a pyridinium, with preference given to an inorganic cation, and more preferably to an alkali metal.

Among the compounds represented by formula (I), preferable ones are compounds represented by the following formula (II), more preferably compounds represented by the following formula (III) or (IV), and particularly preferably compounds represented by the following formula (V):

formula (II)

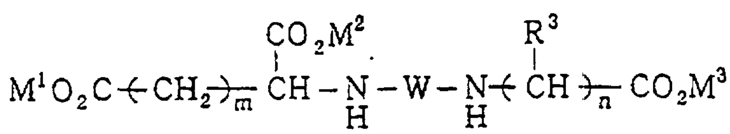

wherein $R^3$, W, m, n, $M^1$, $M^2$, and $M^3$ have the same meanings as those in formula (I), and preferable ones thereof are the same as those preferable in formula (I).

formula (III)

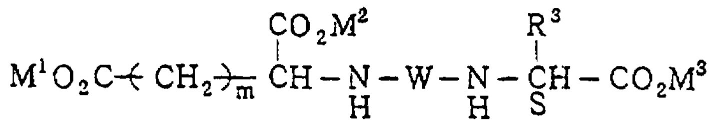

wherein $R^3$, W, m, n, $M^1$, $M^2$, and $M^3$ have the same meanings as those in formula (I), and preferable ones thereof are the same as those preferable in formula (I), and wherein S indicates that the absolute configuration is the S configuration. The asymmetric carbon in which the absolute configuration is not limited, represents any of an S-body, an R-body, or a mixture thereof (e.q. a racemic body).

formula (IV)

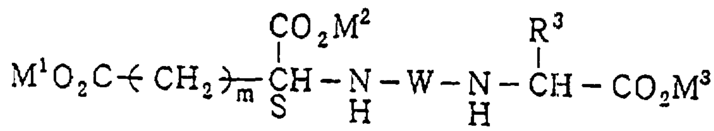

wherein $R^3$, W, m, n, $M^1$, $M^2$, and $M^3$ have the same meanings as those in formula (I), and preferable ones thereof are the same as those preferable in formula (I), and wherein S indicates that the absolute configuration is the S configuration. The asymmetric carbon in which the absolute configuration is not limited, represents any of an S-body, an R-body, or a mixture thereof (e.q. a racemic body).

formula (V)

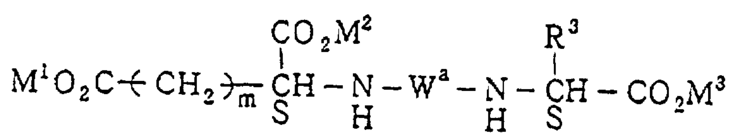

wherein $R^3$, W, m, n, $M^1$, $M^2$, and $M^3$ have the same meanings as those in formula (I), and preferable ones thereof are the same as those preferable in formula (I); S indicates that the absolute configuration is the S configuration, and $W^a$ represents ethylene or trimethylene.

The ethylene or trimethylene represented by $W^a$ may be substituted, and the substituent may be those substituents that may be possessed by the linking group represented by W. Preferably the ethylene or trimethylene represented by $W^a$ is unsubstituted.

Specific examples of the compound represented by formula (I) of the present invention are the following compounds 1 to 8, 11 to 36, 38 and 39, but the present invention is not limited to them. In the following specific examples, those having no indication on the absolute configuration of asymmetric carbons each represent any of an S-body or an R-body or a mixture of them (e.q. a racemic body).

1

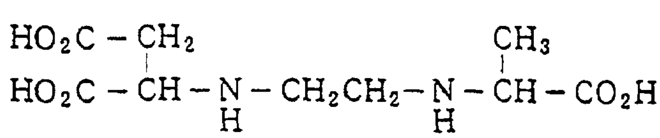

2

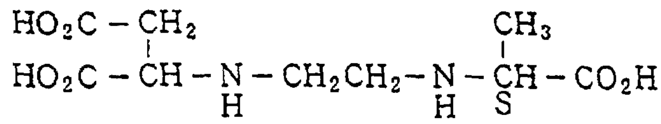

3

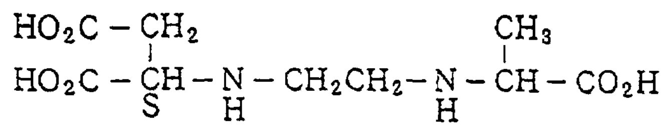

4

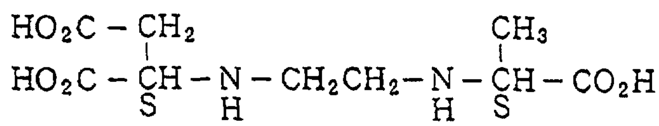

5

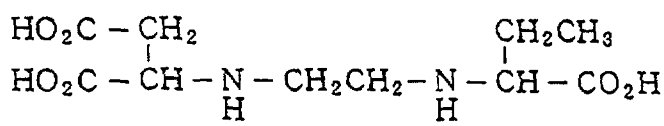

6

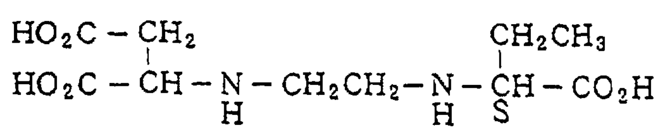

7

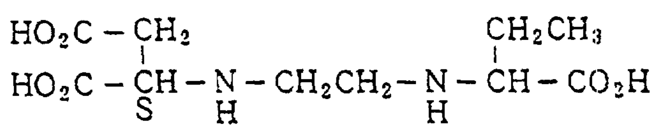

8

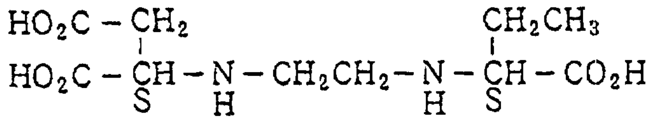

9

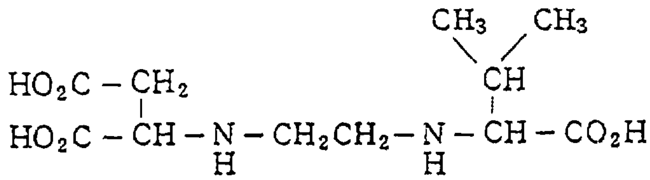

10

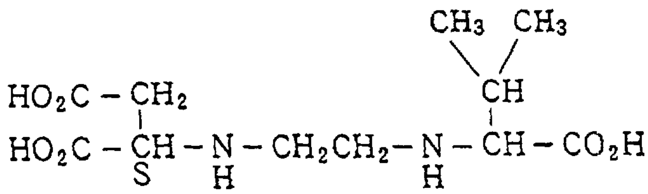

11

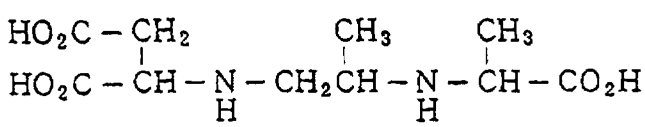

12

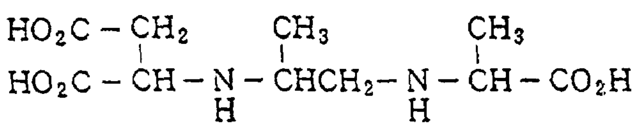

13

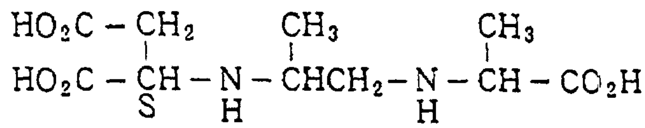

14

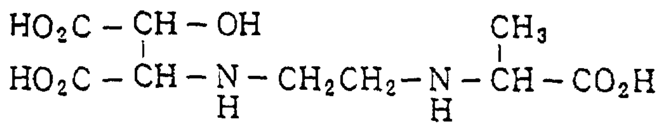

15

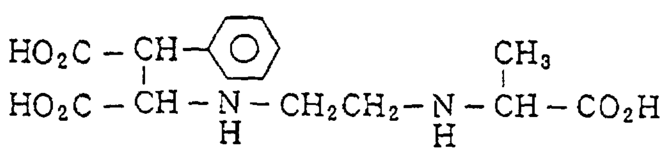

16

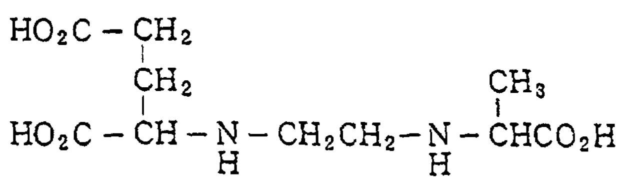

17

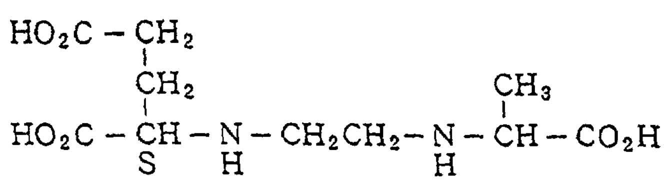

18

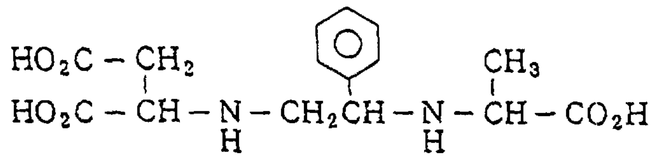

19

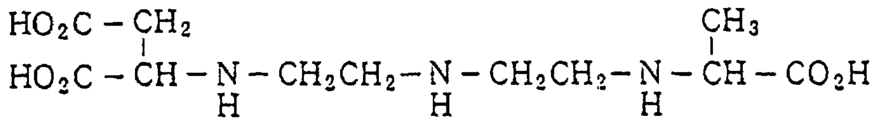

20

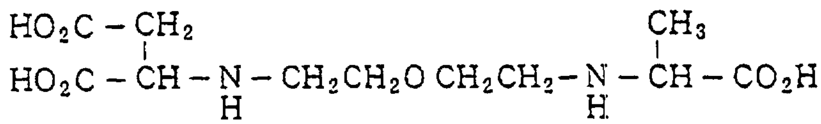

21

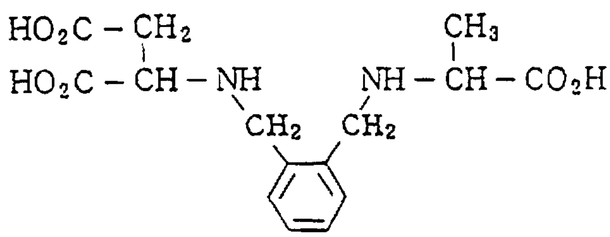

22

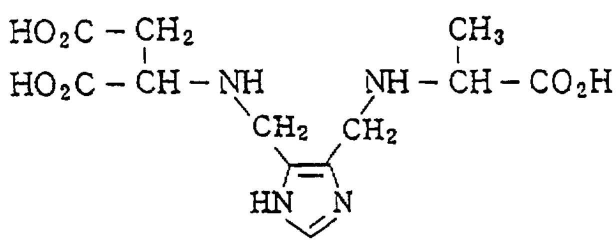

23

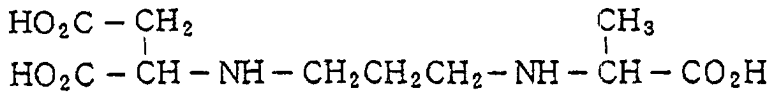

24

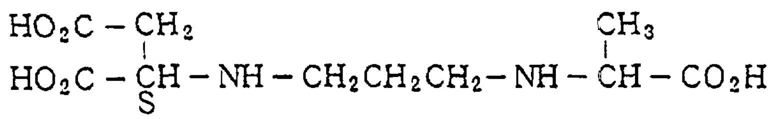

25

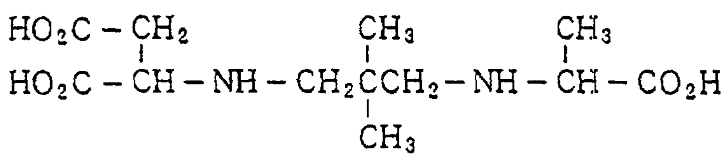

26

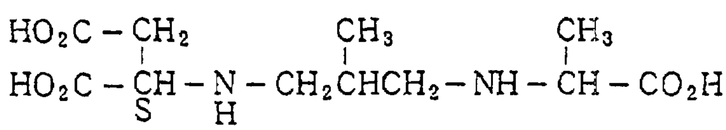

27

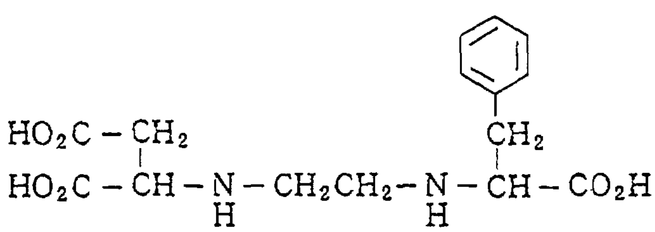

28

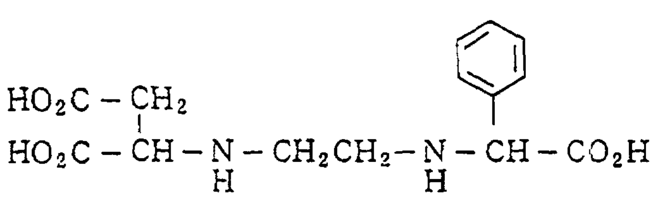

29

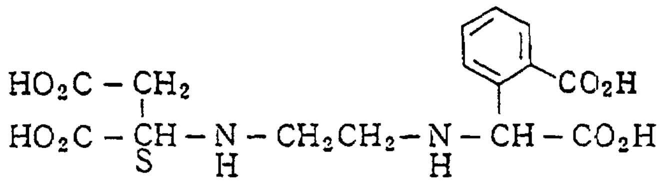

30

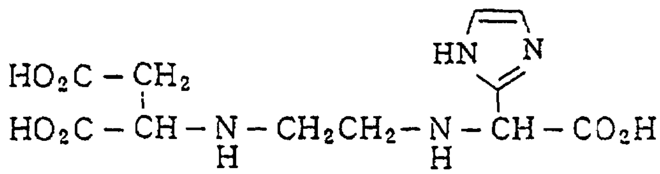

31

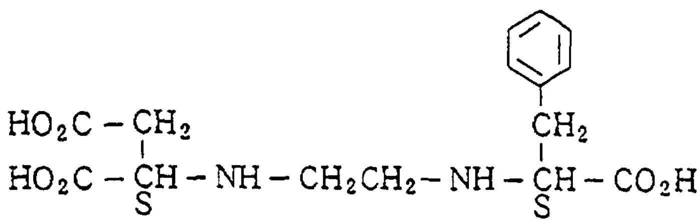

32

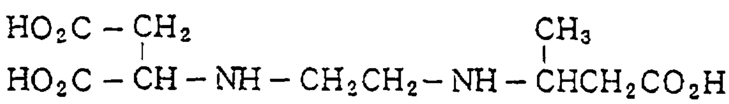

33

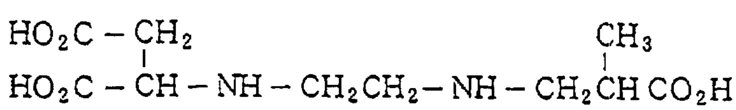

34

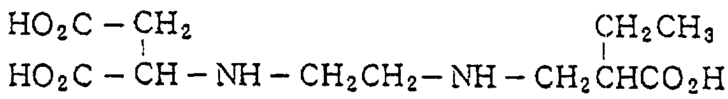

35

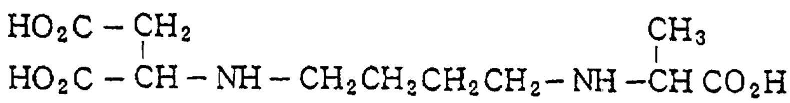

36

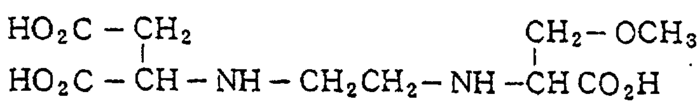

37

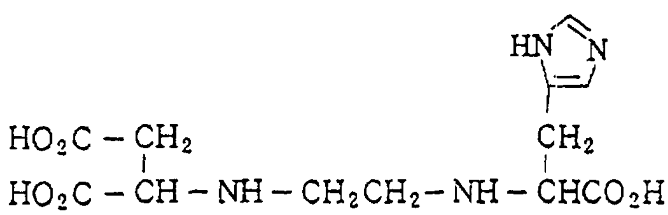

38

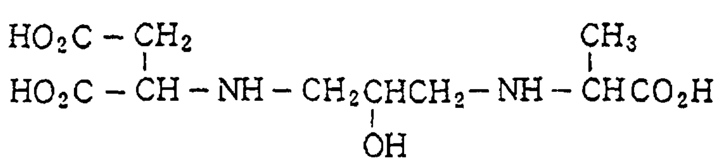

39

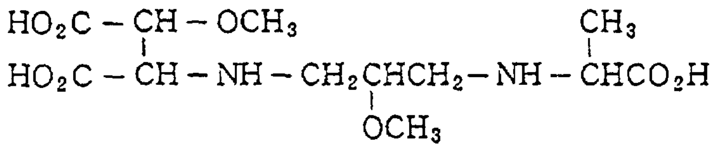

The compound represented by formula (I) can be synthesized according to the scheme shown below. However, they are not to be construed as limiting the synthesis method.

Scheme 1

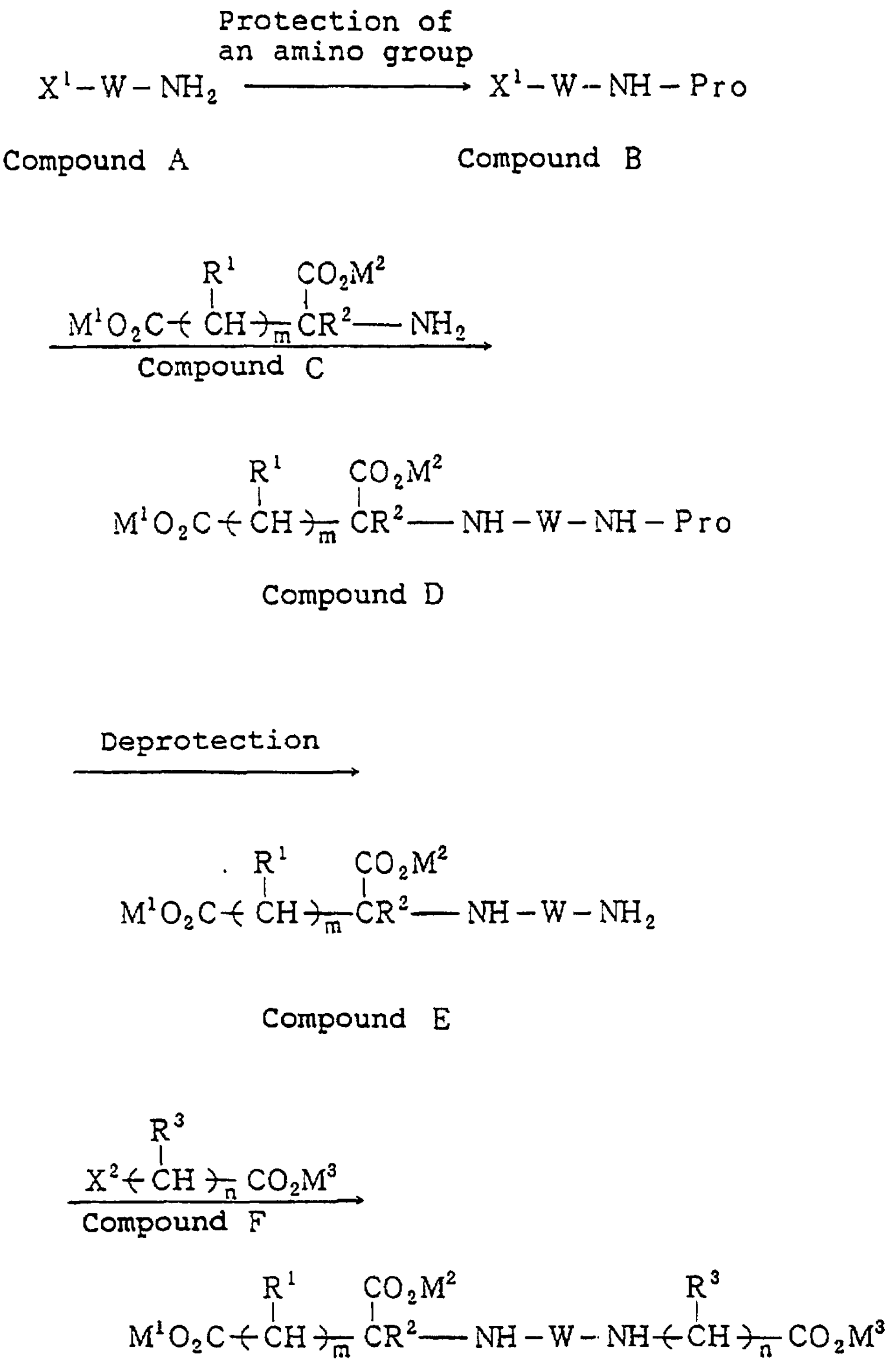


Compound A

Compound B

Compound C

Compound D

Compound E

Compound F

formula (I)

In the scheme, $X^1$ and $X^2$ each represent an elimination group (e.g. a halogen atom, such as fluorine, chlorine, bromine, and iodine, and a sulfonato group, such as methylsulfonato and p-toluenesulfonato); Pro represents a protective group; and $R^1$, $R^2$, $R^3$, m, n, $M^1$, $M^2$, $M^3$, and W have the same meanings as those of $R^1$, $R^2$, $R^3$, m, n, $M^1$, $M^2$,

$M^3$, and W in formula (I), respectively.

When the compound represented by formula (I) is Compound I or Compound J, the compound can be synthesized by the method of Scheme 2.

Scheme 2

Compound F

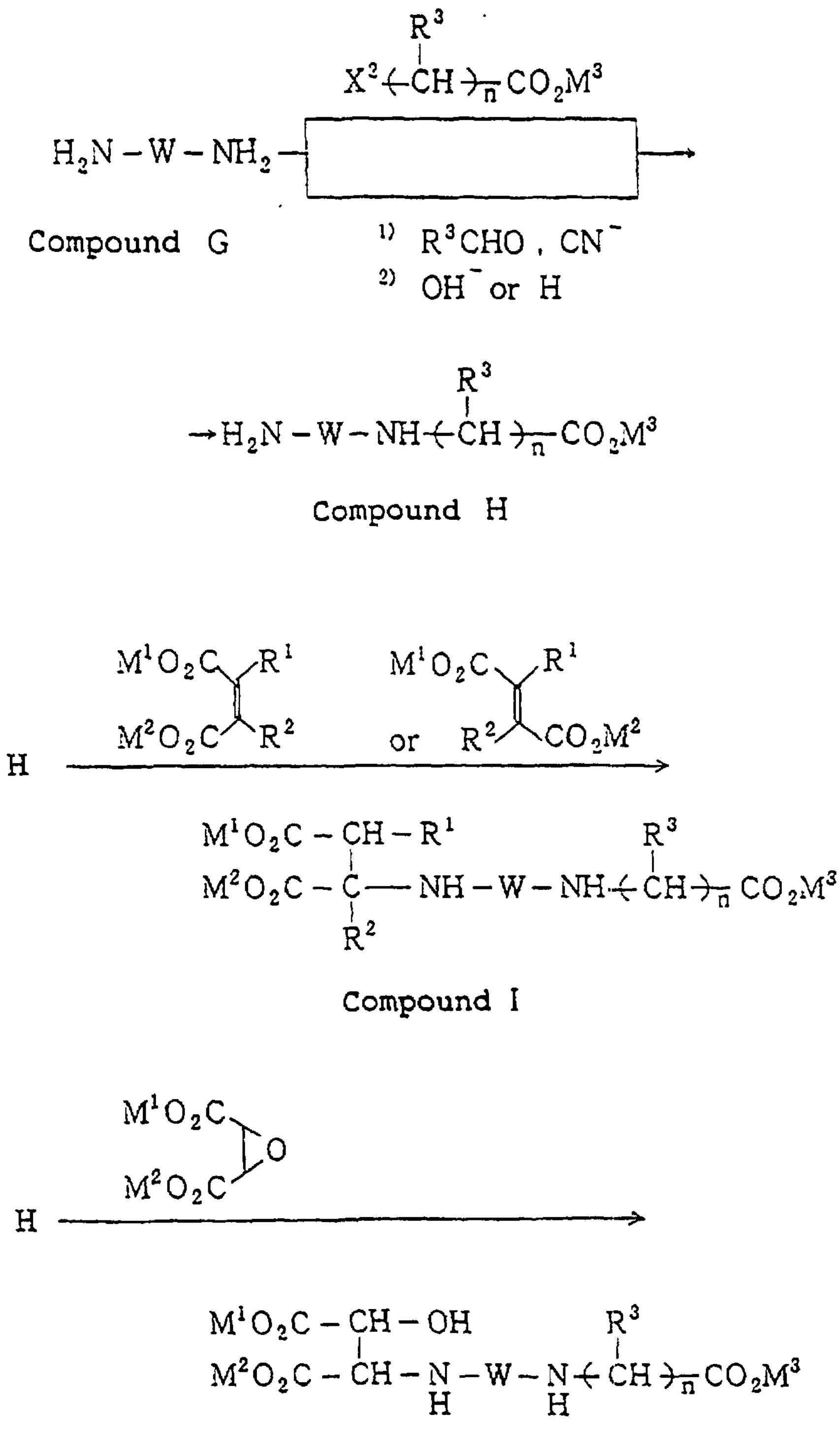


Compound G

Compound H

Compound I

Compound J

In the scheme, $X^2$ represents an elimination group (e.g. a halogen atom, such as fluorine, chlorine, bromine, and iodine, and a sulfonato group, such as methylsulfonato and p-toluenesulfonato); and $R^1$, $R^2$, $R^3$, n, $M^1$, $M^2$, $M^3$,

and W have the same meanings as those of $R^1$, $R^2$, $R^3$, n, $M^1$, $M^2$, $M^3$, and W in formula (I), respectively.) Both the above Compound I and Compound J are compounds included in formula (I).

As the amine derivative (Compound A) and the amino acid derivative (Compound C) that are raw materials in Scheme 1, commercially available compounds can be used.

As the protective group represented by Pro, any group that will not be removed during the reaction can be used, and protective groups described, for example, by Green, Wuts in "Protective Group in Organic Synthesis," Second Edition, pages 309 to 385, John Wiley & Sons, Inc., can be employed. Examples are N-acetyl, N-allyl, N-benzyl, N-benzoyl, benzyl carbamate, t-butyl carbamate, methyl carbamate, N-benzilidene, N-acrylvinyl, N-benzenesulphenyl, N-benzenesulfonyl, N-trimethylsilyl, N-diphenylphosphinyl, N-nitro, and N-borane. Most of these protective groups are commercially available, and they can be used.

Further, as the halogen-substituted alkylcarboxylic acid (Compound F) that is the raw material, commercially available compounds can be used.

As each of the diamine derivative (Compound G), the halogen-substituted alkylcarboxylic acid (Compound F), the aldehyde derivative, the maleic acid derivative, the fumaric acid derivative, and the cyanate, which are the raw materials in Scheme 2, commercially available compounds can be employed.

Further, 2,3-oxiranecarboxylic acid can be synthesized by referring to "Journal of Organic Chemistry," Vol. 28, page 1148 (1963).

The reaction of the halogen-substituted alkylamine (Compound B) with the amino acid (Compound C) can be carried out by referring to pages 302 to 304 of Chelate Chemistry (Kireto Kagaku) (5), written by Kagehira Ueno (Nankodo). The reaction is carried out generally in a solvent at 0 to 150 °C, and preferably at 50 °C or higher. The solvent usually used therein is not particularly limited, unless it takes part in the reaction, and examples of the solvent include water, alcohols (e.g. methanol, ethanol, 2-propanol, and butanol), dimethylformamide, dimethylacetamide, and ethers (e.g. dioxane and tetrahydrofuran). Preferably the reaction is performed in the presence of a base, and examples of the base include an alkali (e.g. sodium hydroxide, potassium hydroxide, sodium carbonate, and potassium carbonate) and a tertiary amine (e.g. triethylamine). If water is used as the solvent, preferably the pH of the reaction conditions is 7 to 12, more preferably 7 to 11, and particularly preferably 8 to 11.

Deprotection is effected under the conditions suitable for the particular involved protective group (described by Green, Wuts, "Protective Groups in Organic Synthesis," Second Edition, pages 309 to 385, John Wiley & Sons, Inc.).

The reaction with the halogen-substituted alkylcarboxylic acid (Compound F) can be carried out by referring to pages 302 to 304 of Chelate Chemistry (Kireto Kagaku) (5), written by Kagehira Ueno (Nankodo). This reaction is generally carried out in a solvent at 0 to 100 °C, and preferably at 90 °C or below. The solvent usually used herein is not particularly limited, unless it takes part in the reaction. The reaction is advantageously carried out by using water or an alcohol (e.g. methanol, ethanol, 2-propanol, and butanol). Preferably the reaction is performed in the presence of a base, and examples of the base include an alkali (e.g. sodium hydroxide, potassium hydroxide, sodium carbonate, and potassium carbonate) and a tertiary amine (e.g. triethylamine). If water is used as the solvent, preferably the pH of the reaction conditions is 7 to 12, more preferably 7 to 11, and further preferably 8 to 11. To introduce a carboxymethyl group (including a substituted carboxymethyl), the Strecker method (see Jikken Kagaku-koza, 4th edition, Vol. 22, pages 193 to 195), known as an amino acid synthesis method, can be used.

The reaction with the halogen-substituted alkylcarboxylic acid (Compound F) in Scheme 2 is carried out under the same conditions as those of the above (Scheme 1), except that the ratio of the amount of the diamine of the raw material to the amount of the halogen-substituted alkylcarboxylic acid is preferably from 1 : 1 to 20 : 1, more preferably from 1 : 1 to 10 : 1, and further preferably from 2 : 1 to 10 : 1.

The synthesis using the Strecker method, wherein an aldehyde and $CN^-$ are used, can be carried out by referring to Jikken Kagaku-koza, 4th edition, Vol. 22, pages 193 to 195. In this case, also the ratio of amount of the diamine of the raw material is preferably in a 1- to 20-fold excess, more preferably a 1- to 10-fold excess, and further preferably a 2- to 10-fold excess.

The reaction (Michael reaction) of the diamine derivative (Compound H) with the olefin derivative (e.g. a maleic acid derivative and a fumaric acid derivative) can be carried out by refering to JP-A-63-199295 and JP-A-3-173857. The solvent used therein is not particularly limited, unless it takes part in the reaction, and examples of the solvent include water, an alcohol (e.g. methanol, ethanol, 2-propanol, and butanol), dimethylformamide, dimethylacetamide, and an ether (e.g. dioxane and tetrahydrofuran). Preferably the reaction is performed in the presence of a base, and examples of the base include an alkali (e.g. sodium hydroxide, potassium hydroxide, sodium carbonate, and potassium carbonate) and a tertiary amine (e.g. triethylamine). If water is used as the solvent, preferably the pH of the reaction conditions is 7 or higher, and more preferably 8 or higher.

Typical synthetic examples of the compounds represented by formula (I) of the present invention are shown below:

Synthetic Example 1 (Synthetic Method of Compound 1)

420 g (6.99 mol) of ethylenediamine and 500 ml of water were placed in a 2-1 three-necked flask, and the internal temperature was dropped to 20 °C using an ice bath. With the internal temperature kept at 20 °C or below, a solution formed by combining 107 g (0.699 mol) of 2-bromopropionic acid and 50 ml of water was added, dropwise. Further, a solution of 56 g of sodium hydroxide in 100 ml of water was added, dropwise, and after stirring well, the mixture was allowed to stand overnight. To the reaction liquid was added a solution of 20 g of sodium hydroxide in 30 ml of water, and the solvent and the excess ethylenediamine were distilled off under reduced pressure using an evaporator. 300 ml of water was added to the residue, and then it was distilled off under reduced pressure. This procedure was repeated three times. Thereafter, distillation was carried out under reduced pressure using a vacuum pump.

To the resulting residue were added 500 ml of water and 89 g (0.769 mol) of maleic acid, followed by stirring well. To the obtained solution was added a solution of 61 g of sodium hydroxide in 60 ml of water, followed by heating under reflux for 2 hours using a hot water bath. Further, 50 g (0.431 mol) of maleic acid and a solution of 34 g of sodium hydroxide dissolved in 40 ml of water were added, followed by heating under reflux for 2 hours. After cooling the mixture to room temperature, the pH was adjusted to 7 with concentrated hydrochloric acid. The reaction liquid was filtered and then was subjected to electrodialysis, to eliminate the salt. Further, the pH was adjusted to 2 with concentrated hydrochloric acid, to desalt. The obtained mixture was concentrated under reduced pressure and was allowed to stand for one day at room temperature, to obtain crystals of the intended compound. The obtained amount was 91 g (overall yield, 53%, based on 2-bromopropionic acid).

Melting point: 209 to 210 °C (decomposed)

| Elementary analysis for $C_9H_{16}N_2O_6$ = 248.24 | | | |
|---|---|---|---|
| | H (%) | C (%) | N (%) |
| Calculated | 6.50 | 43.55 | 11.29 |
| Found | 6.47 | 43.50 | 11.22 |

Synthetic Example 2 (Synthetic Method of Compound 28)

140 g of sodium hydroxide and 500 ml of water were placed in a 2-1 three-necked flask, and after it was cooled to 20 °C on an ice bath, 420 g (6.99 mol) of ethylenediamine was added. With the internal temperature kept at 20 °C, 34.3 g (0.699 mol) of potassium cyanide was added, further 74.2 g (0.699 mol) of benzaldehyde was added, dropwise. After the mixture was allowed to stand 3 days at room temperature, the solvent and the excess ethylenediamine were distilled off under reduced pressure using an evaporator. 300 ml of water was added to the residue, and then it was distilled off under reduced pressure. This procedure was repeated three times. Thereafter, distillation was carried out under reduced pressure using a vacuum pump.

To the resulting residue were added 500 ml of water and 89 g (0.769 mol) of maleic acid, followed by heating under reflux for 2 hours on a hot water bath. Further, 50 g (0.431 mol) of maleic acid and a solution of 34 g of sodium hydroxide in 40 ml of water were added, followed by heating under reflux for 2 hours. After cooling the mixture to room temperature, the pH was adjusted to 7 with concentrated hydrochloric acid. The reaction liquid was filtered and then was subjected to electrodialysis, to desalt. Further, the pH was adjusted to 2 with concentrated hydrochloric acid, to remove the salt. The obtained mixture was concentrated under reduced pressure, to obtain crystals of the intended compound. The obtained amount was 134 g (overall yield, 61%, based on benzaldehyde).

| Elementary analysis for $C_{14}H_{18}N_2O_6$ = 310.31 | | | |
|---|---|---|---|
| | H (%) | C (%) | N (%) |
| Calculated | 5.85 | 54.19 | 9.03 |
| Found | 5.80 | 53.98 | 8.88 |

Synthetic Example 3 (Compound 3)

522 g (2.55 mol) of 2-aminoethylbromide hydrobromide and dichloromethane were placed in a 3-1 three-necked flask, and they were cooled to 10 °C or below using an ice bath. With stirring well, to keep the temperature at 20 °C or below, 500 g (2.93 mol) of Z-chloride and 554 g (5.48 mol) of triethylamine were added, simultaneously, dropwise. Water was added, to extract the salt, and the organic layer was separated. The organic layer was dried over magnesium sulfate, followed by filtering, and the solvent (dichloromethane) was distilled off under reduced pressure.

100 g (0.206 mol) of an aspartic acid dibenzyl ester p-toluenesulfonate and 300 ml of dimethylacetamide were placed in a 1-1 three-necked flask, and then 57 g (0.412 mol) of potassium carbonate and 53 g (0.206 mol) of the N-Z-aminoethylbromide synthesized above were added thereto, followed by heating for 8 hours at 100 °C. After cooling to room temperature, the mixture was filtered, and 500 ml of water and 1,000 ml of dichloromethane were added, followed by stirring well. The layers were separated, and 500 ml of an aqueous dilute hydrochloric acid solution was added to the organic layer, followed by stirring well. After this procedure was repeated three times, the organic layer was dried over magnesium sulfate, and the solvent was distilled off under reduced pressure. The residue was purified by silica gel column chromatography (dichloromethane/methanol) and hydrogenated in an autoclave (pressure, 50 kg/cm$^2$; catalyst, 10% Pd/C; and solvent, water-acetic acid-ethanol), for deprotection. The obtained concentrate was transferred into a three-necked flask, and 50 ml of water and 41 g of sodium hydroxide were added, followed by stirring well. With the temperature kept at 20 °C or below using an ice bath, 31.5 g (0.206 mol) of 2-bromopropionic acid was added, dropwise, thereto. After allowing it to stand overnight, the pH was adjusted to 7 with concentrated hydrochloric acid, to desalt. The pH was further adjusted to 2 with concentrated hydrochloric acid, to desalt, followed by concentration. By allowing the concentrate to stand overnight, crystals of the intended compound were obtained. The obtained amount was 22 g (yield, 42%, based on the aspartic acid dibenzyl ester p-toluenesulfonate).

| Elementary analysis for $C_9H_{16}N_2O_6$ = 248.24 | | | |
|---|---|---|---|
| | H (%) | C (%) | N (%) |
| Calculated | 6.50 | 43.55 | 11.29 |
| Found | 6.30 | 43.27 | 11.08 |

Synthetic Example 4 (Synthesis of Compound 17)

110 g (0.537 mol) of 2-bromoethylamine hydrobromide and 1000 ml of dichloromethane were placed in a 2-1 three-necked flask, and they were cooled to 10 °C or below using an ice bath. With the internal temperature kept at 10 °C or below, 91.6 g (0.537 mol) of Z-chloride was added, dropwise. After stirring for 1 hour, the solvent was distilled off under reduced pressure, and the residue was purified on a silica gel column chromatography (dichloromethane), to give N-Z-2-bromoethylamine (Compound 17a).

49.7 g (0.193 mol) of Compound 17a, 92.3 g (0.385 mol) of diethyl L-glutamate monohydrochloride, 64.7 g (0.770 mol) of sodium hydrogencarbonate and 500 mol of acetonitril were placed in a 1-1 three-necked flask, followed by heating under reflux for 6 hours. The resulting reaction liquid was filtered, and the solvent was distilled off under reduced pressure; then, the residue was purified on a silica gel column chromatography (dichloromethane), to give diethyl 2-(2-benzoylcarboxyaminoethylamino)-pentanediacetate (Compound 17b). 36.3 g (0.095 mol) of Compound 17b, 300 ml of ethanol and 300 ml of water were placed in a 1-L three-necked flask, and they were cooled to 10 °C or below on an ice bath. To the resultant liquid, an aqueous solution of 22 g (0.55 mol) of sodium hydroxide in 100 ml of water was added, slowly. The temperature of the resulting mixture was brought back to room temperature, followed by stirring for 1 hour; the resulting mixture was subjected to hydrogenation in an autoclave (pressure, 50 kg/cm$^2$; catalyst, 10% Pd/C), to carry out deprotection. After Celite filtration, the solvent was distilled off under reduced pressure, to give disodium 2-(2-amino-ethylamino)-pentanediacetate (Compound 17c). All of the obtained Compound 17c was transferred into a 200 ml three-necked flask; and, to the flask, 50 ml of water and 5.0 g (0.125 mol) of sodium hydroxide was added, followed by stirring well while cooling on an ice bath. With the internal temperature kept at 20 °C or below, an aqueous solution of 16.0 g (0.105 mol) of 2-bromopropionic acid that had previously been neutralized with a 50% aqueous sodium hydroxide solution, was added, dropwise, slowly, thereto. After allowing it to stand overnight, the pH was adjusted to 2 with concentrated hydrochloric acid, to desalt using an electrodialysis apparatus. The resulting mixture was subjected to concentration of the solvent under reduced pressure. By allowing the concentrate to stand for 1 month in a refrigerator, crystals of the intended. Compound 17 were obtained. The obtained amount was 15.1 g (yield, 30%, based on Compound 17a).

| Elementary analysis for $C_{10}H_{18}N_2O_6$ = 262.26 | | | |
|---|---|---|---|
| | H (%) | C (%) | N (%) |
| Calculated | 6.92 | 45.80 | 10.68 |
| Found | 7.10 | 45.66 | 10.59 |

Other compounds can also be synthesized in similar way.

The metal salt constituting the heavy metal chelate compound of the compound represented by formula (I)

of the present invention (hereinafter sometimes referred simply to as the heavy metal chelate compound of the present invention) may be Fe(III) (e.g. ferric sulfate, ferric chloride, ferric nitrate, ammonium ferric sulfate, and ferric phosphate), besides, including, salts of Mn(III), Co(III), Rh(II), Rh(III), Au(II), Au(III), Ce(IV). In the present invention, the term "heavy metal" means transition elements (transition metals) and lanthanoid, that is, it means elements of 3A to 7A, 8, 1B and 2B groups in the periodic table, and lanthanoid elements. The heavy metal is preferably transition metals (elements that belong to 3A to 7A, 8, and 1B to 2B groups in the periodic table), more preferably transition metals belonging to 7A, 8, and 1B groups, further preferably Fe(III), Co(III) and Mn(III), still further preferably Fe(III) and Co(III), and most preferably Fe(III).

The heavy metal chelate compound of the present invention may be used in an isolated form as a heavy metal chelate compound, or it may be used in such a way that the above compound represented by formula (I) is reacted with a salt of the above metals in solution. Similarly, it may be used in such a way that the ammonium salt or the alkali metal salt (e.g. the lithium salt, the sodium salt, and the potassium salt) of the compound represented by formula (I) is reacted with a salt of the above metals in solution.

The compound represented by formula (I) is used in an amount of 1.0 mol or more per mol of the metal ion. If the stability of the metal chelate compound is low, the greater the molar amount is, the more preferable it is. Generally the amount of the compound represented by formula (I) used per mol of the metal ion is in the range of 1 to 30 mol.

Specific examples of the heavy metal chelate compound include the following compounds, but the present invention is not limited to them.

1 − a

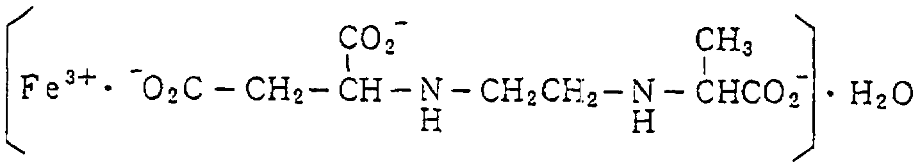

29 − a

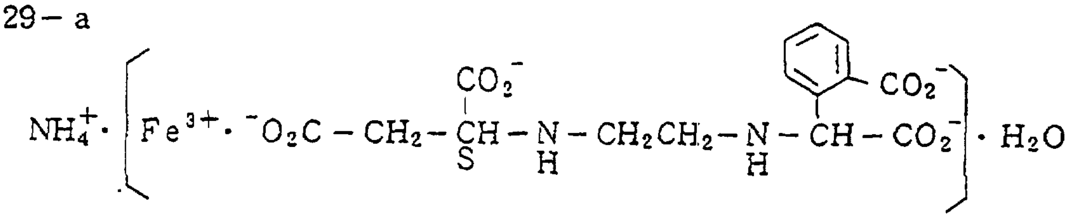

28 − a

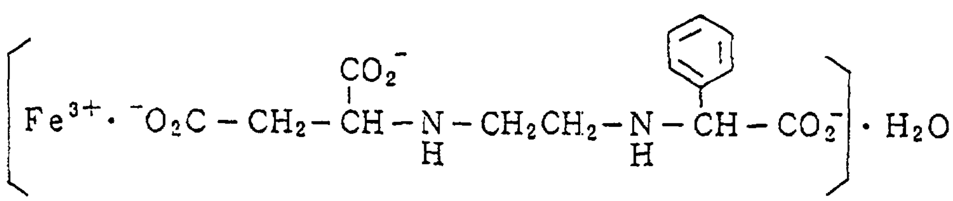

When the metal ion of the heavy metal chelate compound of the present invention is iron, the heavy metal chelate compound can be synthesized by reacting aminopolycarboxylic acids, represented by formula(I), with iron salts. Examples of the iron salts that will be reacted with the aminopolycarboxylic acids represented by formula (I) include ferric sulfate, ferric chloride, ferric nitrate, ammonium ferric sulfate, ferric phosphate, ferric oxide salt, and tri-iron tetroxide. Further, after reacting with a ferrous salt, the compound may be oxidized into the ferric complex. In that case, the oxidation method is not particularly restricted, and, for example, air, oxygen gas, or hydrogen peroxide can be used.

Further, in the synthesis of the iron complex, the free form of the compound represented by formula (I) may be used for the synthesis, or the ammonium salt or the alkali metal salt (e.g. the lithium salt, the sodium salt, and the potassium salt) of the compound represented by formula (I) and a salt of the above metals may be reacted in solution.

The compound represented by formula (I) is used in an amount of 1.0 mol or more per mol of the metal ion. If the stability of the metal chelate compound is low, the greater the molar amount is, the more preferable it is. Generally the amount of the compound represented by formula (I) used per mol of the metal ion is in the range of 1 to 30 mol.

The solvent used therein is not particularly limited, unless it takes part in the reaction, and examples of the solvent include water, an alcohol (e.g. methanol, ethanol, 2-propanol, butanol, and pentanol), dioxane, and dimethylformamide, with preference given to water and an alcohol-series solvent, and particularly to water.

In this reaction, a base (e.g. aqueous ammonia, sodium hydroxide, potassium hydroxide, sodium hydrogencarbonate, potassium hydrogencarbonate, sodium carbonate, and potassium carbonate) may be used to dissolve the aminopolycarboxylic acids represented by formula (I) that are ligands, and the iron complexes obtained therefrom.

The iron complexes may be at any concentration when they are synthesized, as long as they are dissolved. Preferably the concentration is 0.01 to 5 M, and more preferably 0.01 to 3 M.

The reaction can be carried out at a temperature of generally 0 to 130 °C, preferably 0 to 100 °C, and more preferably 10 to 80 °C. The isolation can be carried out at a temperature of generally -20 to 100 °C, preferably -15 to 50 °C, and more preferably -15 to 30 °C.

The isolation of the iron complex of the present invention can be carried out in a usual manner, and the adjustment of the pH is particularly important. If the pH is too low, a stable complex is difficult to be produced, while if the pH is too high, a highly soluble hydroxo complex or a slightly soluble iron hydroxide is apt to be produced, making isolation of the intended iron complex difficult. From this point of view, the synthesis of the iron complex of the present invention can be carried out at a pH of generally 0 to 12, preferably 1 to 10, and more preferably 1 to 7. The adjustment of the pH at that time may be carried out by using an acid (e.g. nitric acid, sulfuric acid, and hydrochloric acid) or a base (e.g. aqueous ammonia, sodium hydroxide, potassium hydroxide, sodium hydrogencarbonate, potassium hydrogencarbonate, sodium carbonate, and potassium carbonate).

Representative synthetic examples of the heavy metal chelate compounds of the present invention are shown below.

Synthetic Example 5 (Compound 1-a)

50.0 g (0.201 mol) of Compound 1 of the present invention and 50 ml of water were placed in a beaker, and ammonia water was added, with stirring well, until Compound 1 was completely dissolved. A solution of 80.8 g (0.200 mol) of iron nitrate nonahydrate in 80 ml of water was added to the above resulting aqueous solution. After the pH was adjusted to 4 with aqueous ammonia, the mixture was filtered, and desalting was carried out by electrodialysis. After the pH of the resulting solution was adjusted to 3.5 with nitric acid, the solution was condensed under reduced pressure and stored in a refrigerator. After one month, the deposited crystals were filtered, washed with a small amount of cold water, and dried at room temperature. Thus, 26.9 g of the intended Compound 1-a was obtained. Yield 42.1%.

| Elementary analysis for $C_9H_{15}N_2FeO_7$ = 319.07 | | | |
|---|---|---|---|
| | H (%) | C (%) | N (%) |
| Calculated | 4.74 | 33.88 | 8.78 |
| Found | 4.85 | 33.79 | 8.65 |

The compound represented by formula (I) according to the present invention may be contained, as a silver-halide-photographic additive that does not adversely affect photographic properties (e.g. sensitivity and fog), into a light-sensitive material (e.g. a photographic constitutional layer, such as a photographic emulsion layer and an intermediate layer), or it may be contained into a processing composition for a light-sensitive material.

By using the above compound, the actions of a chelating agent, a bleaching agent, an oxidizing agent, a precipitation-preventing agent, a stain inhibitor, a stabilizer, a reducing agent, can be obtained.

The heavy metal chelate compound of the present invention has the effect of oxidizing agents for silver halide photographic light-sensitive materials (particularly the effect of bleaching agents for color light-sensitive materials).

According to a preferable mode of a processing composition containing the heavy metal chelate compound of the present invention, after a silver halide color photographic light-sensitive material that has been exposed imagewise is subjected to color-development, the silver halide color photographic light-sensitive material is, at least, processed with a processing solution having a bleaching capacity and containing the heavy metal chelate compound of the present invention as a bleaching agent, so that the developed silver is bleached quite quickly. In that mode, occurrence of a precipitate, dirt on the light-sensitive material surface, and clogging of a filter at the time of running processing that will be observed in the case of a conventional bleaching agent that can perform quick bleaching, are less.

In passing, the present invention is an invention characterized in an oxidizing agent in a photographic processing composition, and particularly in a bleaching agent in a processing composition having a bleaching capacity for color light-sensitive materials. Other requirements for materials and the like can be suitably selected from those requirements generally applied to materials.

Hereinbelow, the processing compositions (processing solutions) containing the heavy metal chelate compound and the chelating agent of the present invention will be described. The compositions may be in the form of a processing solution as well as a solid (e.g. a powder or granules), a paste.

The heavy metal chelate compound of the present invention may be contained in any processing solution (e.g. a bleach-fix solution, a fixing solution, an intermediate bath between a color development step and a desilvering step, or a stabilizing solution). It is particularly effective as a reduction solution for black-and-white light-sensitive materials and a processing solution having a bleaching capacity for color light-sensitive materials (bleaching solution or bleach-fix solution), wherein the heavy metal chelate compound of the present invention is generally contained in an amount of 0.005 to 1 mol per liter of the processing solution.

Hereinbelow, processing solutions having a bleaching capacity that are preferable modes will be described. As described above, the heavy metal chelate compound of the present invention is effective as a bleaching agent in a processing solution having a bleaching capacity and containing the heavy metal chelate compound of the present invention in an amount of generally 0.005 to 1 mol, preferably 0.01 to 0.5 mol, and particularly preferably 0.05 to 0.5 mol, per liter of the processing solution. Parenthetically, the heavy metal chelate compound of the present invention can exhibit its excellent performance even if it is used in a dilute concentration of generally 0.005 to 0.2 mol, preferably 0.01 to 0.2 mol, and more preferably 0.05 to 0.18 mol, per liter of the processing solution.

Further, when the heavy metal chelate compound is added to a processing solution having a bleaching capacity, it may be added not only in the oxidized form (e.g. a chelate compound of Fe (III)) but also in the reduced form (e.g. a chelate compound of Fe(II)).

When the heavy metal chelate compound of the present invention is used as a bleaching agent in a processing solution having a bleaching capacity, it may be used in combination with another bleaching agent in an amount whereby the effect of the present invention is exhibited (preferably in the range of 0.01 mol or less, and more preferably 0.005 mol or less, per liter of the processing solution). Examples of the bleaching agent that can be used include compounds of polyvalent metals, such as iron(III), peracids, quinones, and nitro compounds. Examples of the typical bleaching agent include organic complex salts of iron(III), such as iron complex salts of ethylenediaminetetraacetic acid, diethylenetriaminepentaacetic acid, cyclohexandiaminetetraacetic acid, methyliminodiacetic acid, or glycol ether diaminetetraacetic acid; bleaching agents such as 1,3-propylenediaminetetraacetic acid iron complex salt, described in JP-A-4-121739, from page 4, lower right column, to page 5, upper left column; carbamoyl bleaching agents described in JP-A-4-73647; bleaching agents having a heterocycle, described in JP-A-4-174432; bleaching agents described in EP-A-520 457, such as N-(2-carboxyphenyl)iminodiacetic acid ferric complex salt; bleaching agents described in EP-A-530 828 (A1), such as ethylenediamine-N-2-carboxyphenyl-N,N',N'-triacetic acid ferric acetate; bleaching agents described in EP-A-501 479, bleaching agents described in EP-A-567 126, bleaching agents described in JP-A-4-127145, and aminopolycarboxylic acid ferric salts or their salts, as described in JP-A-3-144446, page (11), all of which are not to be construed as limiting the present invention.

Preferably the processing solution having a bleaching capacity that contains the heavy metal chelate compound according to the present invention contains, besides that heavy metal chelate compound as a bleaching agent, a halide, such as a chloride, a bromide, and an iodide, as a rehalogenating agent for facilitating oxidation of silver. Further, in place of the halide, an organic ligand that can form a slightly soluble silver salt, may be added. The halide is added in the form of an alkali metal salt, an ammonium salt, or a salt of guanidine or an amine. Specific examples are sodium bromide, ammonium bromide, potassium chloride, guanidine hydrochloride, potassium bromide, potassium chloride. In the processing solution having a bleaching capacity of the present invention, the amount of the rehalogenating agent is generally suitably 2 mol/liter or less, and in the case of a bleaching solution, the amount is preferably 0.01 to 2.0 mol/liter, more preferably 0.1 to 1.7 mol/liter, and particularly preferably 0.1 to 0.6 mol/liter. In the case of a bleach-fix solution, the amount is preferably 0.001 to 2.0 mol/liter, more preferably 0.001 to 1.0 mol/liter, and particularly preferably 0.001 to 0.5 mol/liter.

Further, to the bleaching solution or the bleach-fix solution according to the present invention, are added a bleaching accelerator, a corrosion inhibitor for preventing a processing bath tank from being corroded, a buffer for keeping the pH of the solution, a fluorescent whitening agent, an antifoaming agent, if necessary.

Examples of the bleaching accelerator to be used include compounds having a mercapto group or a disulfide group, as described in US-A-3 893 858, German patent No. 1 290 821, British patent No. 1 138 842, JP-A-53-95630, and Research Disclosure No. 17129 (July, 1978); thiazolidine derivatives, as described in JP-A-50-140129; thiourea derivatives, as described in US-A-3 706 561; iodides, as described in JP-A-58-16235; polyethylene oxides, as described in German patent No. 2 748 430; and polyamine compounds, as described in JP-B-45-8836. Further, compounds described in US-A-4 552 834 are also preferably used. These bleaching accelerators may be added into a light-sensitive material. These bleaching accelerators are particularly effective when bleach-fixing a color light-sensitive material for shooting (photographing). Particularly preferably, the bleaching accelerators are mercapto compounds described in GB-1 138 842 and JP-A-2-190856.

The pH of the bleaching solution or the bleach-fix solution according to the present invention is generally 2.0 to 8.0, and preferably 3.0 to 7.5. When, immediately after a light-sensitive material for photographing is subjected to color-development, the light-sensitive material is bleached or bleach-fixed, preferably the pH of the solution is 7.0 or below, and more preferably 6.4 or below, in order to suppress bleach fogging. Particularly in the case of the bleaching solution, the pH is preferably 3.0 to 5.0. If the pH is 2.0 or below, the metal chelate of the present invention becomes unstable easily in some cases, and therefore the pH is preferably 2.0 to 6.4. In the case of color print materials, preferably the pH is in the range of 3 to 7.

As the pH buffer for that, any buffer can be used that resists oxidation by a bleaching agent and that has a buffering action in the above pH range. Examples include organic acids, such as acetic acid, glycolic acid, lactic acid, propionic acid, butyric acid, malic acid, chloroacetic acid, levulinic acid, ureidopropionic acid, formic acid, pyruvic acid, isobutyric acid, pivalic acid, aminobutyric acid, valeric acid, isovaleric acid, asparagine, alanine, arginine, ethionine, glycine, glutamine, cysteine, serine, methionine, leucine, histidine, benzoic acid, hydroxybenzoic acid, nicotinic acid, oxalic acid, malonic acid, succinic acid, tartaric acid, maleic acid, fumaric acid, oxaloic acid, glutaric acid, adipic acid, aspartic acid, glutamic acid, cystine, ascorbic acid, phthalic acid, and terephthalic acid; and organic bases, such as pyridine, dimethylpyrazole, 2-methyl-o-oxazoline, aminoacetonitrile, and imidazole. These buffers may be used in combination of two or more of these. In the present invention, an organic acid having an acid dissociation constant (pKa) of 2.0 to 5.5 is preferable, and the organic acid is preferably a dibasic acid. Particularly preferable dibasic acids are, for example, succinic acid, glutaric acid, maleic acid, fumaric acid, malonic acid, and adipic acid. Most preferable dibasic acids are succinic acid, glutaric acid, and maleic acid. These organic acids may be used in the form of alkali metal salts (e.g. lithium salts, sodium salts, and potassium salts) and ammonium salts. The amount of these buffers to be used is suitably generally 3.0 mol or less, preferably 0.1 to 2.0 mol, more preferably 0.2 to 1.8 mol, and particularly preferably 0.4 to 1.5 mol, per liter of the processing solution having a bleaching capacity.

To adjust the pH of the processing solution having a bleaching capacity to the foregoing range, the above acids and alkalis (e.g. aqueous ammonia, KOH, NaOH, potassium carbonate, sodium carbonate, imidazole, monoethanolamine, and diethanolamine) may be used in combination. Among these, aqueous ammonia, KOH, NaOH, potassium carbonate, and sodium carbonate are preferred.

Further, as the corrosion inhibitor, a nitrate is preferably used, such as ammonium nitrate, sodium nitrate, and potassium nitrate. The amount thereof that will be added is generally 0.01 to 2.0 mol per liter, and preferably 0.05 to 0.5 mol per liter.

In recent years, awareness of preservation of the earth's environment has increased, and attempts are being made to decrease nitrogen atoms discharged into the environment. From this point of view, desirably, the processing solution according to the present invention is substantially free from ammonium ions.

Additionally stated, in the present invention, the term "substantially free from ammonium ions" means that the concentration of ammonium ions is generally 0.1 mol/liter or less, preferably 0.08 mol/liter or less, more preferably 0.01 mol/liter or less, and particularly preferably no ammonium ion is contained.

To reduce the concentration of ammonium ions to the range for use in the present invention, as an alternative cation species, an alkali metal ion or an alkaline earth metal ion is preferable, and particularly an alkali metal ion is preferable. Among others, a lithium ion, a sodium ion, and a potassium ion are particularly preferable. Specifically, as bleaching agents, sodium salts or potassium salts of organic acid ferric complexes, and, as rehalogenating agents in the processing solution having a bleaching capacity, potassium bromide and sodium bromide, as well as potassium nitrate and sodium nitrate, can be mentioned as examples.

Further, as an alkali agent used for adjusting the pH, for example, potassium hydroxide, sodium hydroxide, potassium carbonate, and sodium carbonate are preferable.

Particularly preferably, the processing solution having a bleaching capacity according to the present invention is subjected to aeration at the time of the processing, because such aeration keeps photographic properties extremely stable. Various means known in this technical field can be used for the aeration. For example, there are several methods, such as blowing of air into the processing solution having a bleaching capacity, and absorption of air by means of an ejector.

At the time of the blowing of air, it is preferred to deliver air into a solution through a gas-scattering tube having fine pores. The air-scattering tube is widely used for an airing tub in the activated sludge processing. For further particulars about the aeration, the articles described in Z-121, Using Process C-41, Third edition (1982), published by Eastman Kodak Co., pp. BL-1 to BL-2, can be referred to. It is preferred to vigorously stir the processing solution having a bleaching capacity according to the present invention. A method described in JP-A-3-33847, page 8, upper right

column, line 6, to the lower left column, line 2, can be used to accomplish the above-mentioned purpose.

Bleaching or bleach-fixing process can be carried out at a temperature of generally 30 °C to 60 °C, and preferably 35°C to 50°C.

In the case of light-sensitive materials for photographing, the time of the bleaching step and/or the bleach-fix step is in the range of generally 10 sec to 7 min, and preferably 10 sec to 4 min. In the case of printing light-sensitive materials, the time of the bleaching step and/or the bleach-fix step is in the range of generally 5 to 70 sec, preferably 5 to 60 sec, and more preferably 10 to 45 sec. Under these preferable processing conditions, quick good results without increased stain were obtained.

The light-sensitive material processed with a processing solution having a bleaching capacity is fixed or bleach-fixed. Parenthetically, when the processing solution having a bleaching capacity is a bleach-fix solution, thereafter a fixing process or a bleach-fix process may or may not be carried out. The fixing solution or the bleach-fix solution is also preferably one described in JP-A-3-33847, page 6, lower right column, line 16, to page 8, upper left column, line 15.

Parenthetically, as the fixing agent in the desilvering step, ammonium thiosulfate is generally used, but other known fixing agents, such as meso-ionic compounds, thioether compounds, thioureas, large amounts of iodides, or hipo, may substitute for it. These are described, for example, in JP-A-60-61749, JP-A-60-147735, JP-A-64-21444, JP-A-1-201659, JP-A-1-210951, JP-A-2-44355, and US-A-4 378 424. Examples include ammonium thiosulfate, sodium thiosulfate, potassium thiosulfate, guanidine thiosulfate, ammonium thiocyanate, sodium thiocyanate, potassium thiocyanate, dihydroxyethyl thioether, 3,6-dithia-1,8-octanediol, and imidazole. Among these, thiosulfates and meso-ionic compounds are preferable. In view of quick fixability, ammonium thiosulfate is preferable, but if the environmental problem is taken into account as described above to make the processing solution substantially free from ammonium ions, sodium thiosulfate and meso-ionic compounds are further preferable. Furthermore, a combination of two or more fixing agents can be used to perform further quick fixing. For example, a combination of ammonium thiosulfate or sodium thiosulfate with the above ammonium thiocyanate, imidazole, thiourea, a thioether is preferably used. In this case, the second fixing agent is preferably added in an amount in the range of 0.01 to 100 mol% to ammonium thiosulfate or sodium thiosulfate.

The amount of the fixing agent is generally 0.1 to 3.0 mol, and preferably 0.5 to 2.0 mol, per liter of the bleach-fix solution or the fixing solution. The pH of the fixing solution varies depending on the type of the fixing agent, and it is generally 3.0 to 9.0. Particularly, when a thiosulfate is used, the pH of the fixing solution is preferably 5.8 to 8.0, because stable fixing performance can be obtained.

A preservative can be added to the bleach-fix solution or the fixing solution, to increase the stability of the solution with time. In the case of the bleach-fix solution or fixing solution containing a thiosulfate, as a preservative, a sulfite and/or hydroxylamine, hydrazine, a bisulfite adduct of an aldehyde (e.g. a bisulfite adduct of acetaldehyde, and particularly preferably a bisulfite adduct of an aromatic aldehyde, described in JP-A-1-298935) is effective.

Further, preferably the bleach-fix solution containing the heavy metal chelate compound of the present invention contains at least one sulfinic acid and its salt. Preferable examples of the sulfinic acids and their salts include compounds described, for example, in JP-A-1-230039, JP-A-1-224762, JP-A-1-231051, JP-A-1-271748, JP-A-2-91643, JP-A-2-251954, JP-A-2-251955, JP-A-3-55542, JP-A-3-158848, JP-A-4-51237, JP-A-4-329539, US-A-5 108 876, US-A-4 939 072, EP-A-255 722 (A), and EP-A-463 639. Optionally substituted arylsulfinic acids or their salts are more preferable, and optionally substituted phenylsulfinic acids or their salts are further more preferable. Examples of their substituents include an alkyl group having 1 to 4 carbon atoms, an aryl group having 6 to 10 carbon atoms, a carbamoyl group having 1 to 5 carbon atoms, an alkoxycarbonyl group having 1 to 5 carbon atoms, an alkoxy group having 1 to 4 carbon atoms, a sulfinic acid group, a sulfonic acid group, a carboxylic acid group, a hydroxyl group, and a halogen atom.

Hereinbelow, preferable specific examples of sulfinic acids and their salts are shown.

S − 1

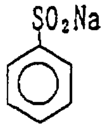

S − 2

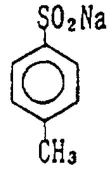

S－3

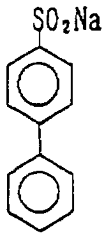

S－4

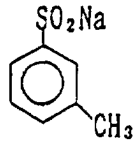

S－5

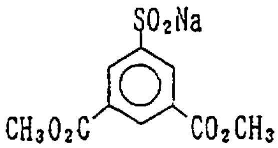

S－6

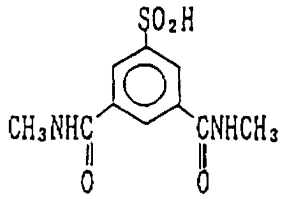

S－7

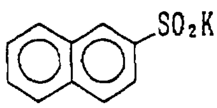

S－8

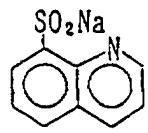

S－9

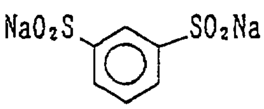

S－10

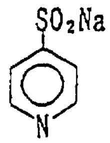

S－11

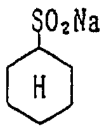

S－12

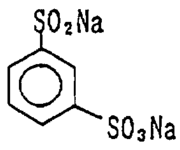

S-13

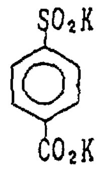

S-14

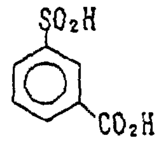

S-15

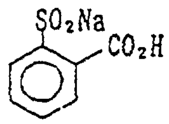

S-16

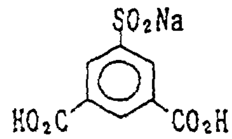

S-17

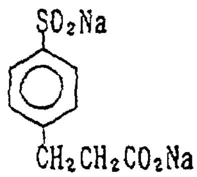

S-18

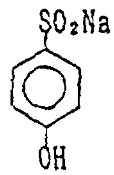

S-19

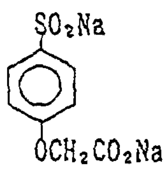

S-20

$^{n}C_4H_9SO_2Na$

S-21

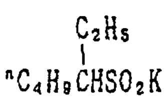

S-22

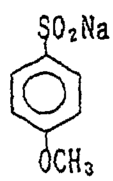

.

S -23

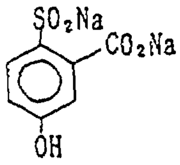

S -24

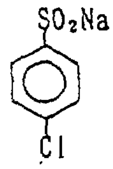

The amount of the sulfinic acid or its salt that will be added to the bleach-fix solution or the fixing solution is generally 1 x $10^{-4}$ to 1 mol, preferably 1 x $10^{-3}$ to 0.5 mol, and more preferably 1 x $10^{-2}$ to 0.1 mol, per liter of the processing solution.
To keep the pH of the bleach-fix solution or the fixing solution constant, the addition of a buffer to the solution is preferable. Examples of the buffer include phosphates; imidazoles, such as imidazole, 1-methylimidazole, 2-methylimidazole, and 1-ethyl-imidazole; triethanolamine, N-allylmorpholine, and N-benzoylpiperazine.
Further, in the fixing solution, various chelating agents can be added to sequester iron ions carried in from the bleaching solution, to improve the stability of the solution. Examples of preferable chelating agents used therefor include, in addition to the compounds of the present invention, 1-hydroxyethylidene-1,1-diphosphonic acid, nitrilotrimethylenephosphonic acid, 2-hydroxy-1,3-diaminopropanetetraacetic acid, ethylenediaminetetraacetic acid, diethylenetriaminepentaacetic acid, ethylenediamine-N-(β-hydroxyethyl)-N,N',N'-triacetic acid, 1,2-diaminopropanetetraacetic acid, 1,3-diaminopropanetetraacetic acid, nitrilotriacetic acid, cyclohexanediaminetetraacetic acid, iminodiacetic acid, dihydroxyethylglycine, ethyl ether diaminetetraacetic acid, glycol ether diaminetetraacetic acid, ethylenediaminetetrapropionic acid, phenylenediaminetetraacetic acid, 1,3-diaminopropanol-N,N,N',N'-tetramethylenephosphonic acid, ethylenediamine-N,N,N',N'-tetramethylenephosphonic acid, 1,3-propylenediamine-N,N,N',N'-tetramethylenephosphonic acid, serine-N,N-diacetic acid, 2-methyl-serine-N,N-diacetic acid, 2-hydroxymethyl-serine-N,N-diacetic acid, hydroxyethyliminodiacetic acid, methyliminodiacetic acid, N-(2-acetamido)-iminodiacetic acid, nitrilotripropionic acid, ethylenediaminediacetic acid, ethylenediaminedipropionic acid, 1,4-diaminobutanetetraacetic acid, 2-methyl-1,3-diaminopropanetetraacetic acid, 2-dimethyl-1,3-diaminopropanetetraacetic acid, alanine, tartaric acid, hydrazidediacetic acid, and N-hydroxy-iminodipropionic acid, and their alkali metal salts (e.g. lithium salts, sodium salts, and potassium salts) and their ammonium salts.
The fixing process can be carried out with temperature of generally 30 to 60 °C, and preferably 35 to 50 °C.
The processing time of the fixing process is, in the case of light-sensitive materials for shooting, in the range of generally 15 sec to 2 min, and preferably in the range of 25 sec to 1 min 40 sec, and in the case of printing light-sensitive material, in the range of generally 8 sec to 80 sec, and preferably in the range of 10 sec to 45 sec.
Generally, the desilverling process is carried out with combination of a bleaching step, a bleach-fixing step and a fixing step. Specific modes thereof are given below.

(1) Bleaching/fixing
(2) Bleaching/bleach-fixing
(3) Bleaching/bleach-fixing/fixing
(4) Bleaching/washing/fixing
(5) Bleach-fixing
(6) Fixing/bleach-fixing

In photographing light-sensitive materials, (1), (2), (3), (4), and (5) are preferable, and in the present invention, processes that include a bleach-fix solution, as in (2), (3), and (5), exhibit remarkable effects, with preference given particularly to (5).
The present invention can be applied to a desilvering process that is carried out via an adjusting bath, a stop bath, a washing bath after the color-development process.
The processing solution according to the present invention, having a bleaching capacity, can be reused in the processing step by recovering the overflow liquid after use, and then compensating for the composition by the addition of components. Such a usage, which is generally called "regeneration," is preferably used in the present invention. With regard to the details of the regeneration, the items described in Fuji Film Processing Manual, Fuji Color Negative Film, CN-16 Process (revised in August 1990), pp 39-40 (published by Fuji Photo Film Co., Ltd.), can be referred to.
The kit for adjusting the processing solution having a bleaching capacity according to the present invention may be in the form of a liquid, a powder, or a solid, and if ammonium salts are eliminated, most of the raw materials

can be supplied in the form of powders, and materials that are less hygroscopic more easily form a powder.

Preferably the above kit for the regeneration is preferably in the form of a powder, in view of the reduction of the amount of waste liquor, because in that case, excess water is not used and it can be directly used.

With regard to the regeneration of the processing solution having a bleaching capacity, in addition to the above described aeration methods, the methods disclosed in Shashin Kogaku no Kiso - Ginn-en Shashin Hen (The Fundamentals of Photographic Technology-Silver Salt Photography) (edited by Nippon Shashin Gakkai, published by Corona, Co., 1979) can be utilized. Specific examples of the regeneration methods of the solution include a regeneration method of a bleaching solution by electrolysis and a regeneration method by a hydrogen peroxide, a bromous acid, ozone making use of a bromic acid, a chlorous acid, a bromine, a bromine precursor, a persulfate, a hydrogen peroxide, and a catalyst.

In the regeneration method by electrolysis, a regeneration processing is carried out by putting an anode and a cathode in the same bleaching bath, or by separating an anode bath from a cathode bath by a diaphragm, as well as that a bleaching solution and a developing solution and/or a fixing solution can be regeneration-processed at the same time, also using a diaphragm.

Regeneration of the fixing solution and the bleach-fixing solution is carried out by an electrolytic reduction of the accumulated silver ion. In addition, the removal of the accumulated halogen ion by means of an anion exchange resin is also preferred, for maintaining the fixing ability.

To reduce the amount of washing water to be used, ion exchange or ultrafiltration can be carried out, with preference given to ultrafiltration.

Incidentally, the present invention can also be applied to a reduction solution for correcting a silver image consisting of dots and/or lines obtained by subjecting to development a photomechanical silver halide light-sensitive material that has been exposed to light.

Further, the chelating agent represented by formula (I) or its heavy metal chelate compound can be applied to all processing compositions for processing silver halide black-and-white light-sensitive materials and silver halide color light-sensitive materials. For silver halide black-and-white light-sensitive materials, the chelating agent represented by formula (I) or its heavy metal chelate compound can be applied, for example, to a general purpose black-and-white developing solution, a contagious developing solution for lithfilms, a fixing solution, and washing water, and for silver halide color light-sensitive materials, the chelating agent represented by formula (I) or its heavy metal chelate compound can be applied, for example, to a color developer, a bleaching solution, a fixing solution, a bleach-fix solution, an adjusting solution, a stop solution, a hardening solution, washing water, a stabilizing solution, a rinsing solution, a fogging solution, and a toning solution, but the present invention is not limited to them. The present invention is effective particularly for a black-and-white developing solution, a color developer, a fixing solution, and a stabilizing solution, and more particularly excellent for a black-and-white developing solution and a color developer.

The amount of the compound represented by formula (I) that will be added varies depending on the processing composition to which the compound is added, and it is generally in the range of 10 mg to 50 g, per liter of the processing composition.

In more detail, for example, when the compound represented by formula (I) is added to a black-and-white developing solution or a color developer, a preferable amount is 0.5 to 10 g per liter of that processing solution; when the compound represented by formula (I) is added to a bleaching solution (comprising, for example, hydrogen peroxide, a persulfate, hydrobromic acid, or the like), the preferable amount is 0.1 to 20 g per liter of that solution; when the compound represented by formula (I) is added to a fixing solution or a bleach-fix solution, the preferable amount is 1 to 40 g per liter of that solution, and when the compound represented by formula (I) is added to a stabilizing bath, the preferable amount is 50 mg to 1 g per liter of that bath.

The compounds represented by formula (I) may be used singly or in the form of a combination of two or more.

In a color developer and a black-and-white developer, the addition of the compound of the present invention can prevent precipitation and can improve the stability of the developers.

As the color developer that can be used in the present invention, can be mentioned those described in JP-A-3-33847, page 9, left upper column, line 6, to page 11, right lower column, line 6, and JP-A-5-197107.

Parenthetically, as the color-developing agent in a color-developing step, known aromatic primary amine color developers can be employed, and preferably p-phenylenediamine-series compounds are used. Representative examples thereof include 3-methyl-4-amino-N,N-diethylaniline, 3-methyl-4-amino-N-ethyl-N-β-hydroxyethylaminline, 3-methyl-4-amino-N-ethyl-N-β-methanesulfoneamidoethylaniline, 3-methyl-4-amino-N-ethyl-β-methoxyethylaniline, 4-amino-3-methyl-N-methyl-N-(3-hydroxypropyl)aniline, 4-amino-3-methyl-N-ethyl-N-(3-hydroxypropyl)aniline, 4-amino-3-methyl-N-ethyl-N-(2-hydroxypropyl)aniline, 4-amino-3-ethyl-N-ethyl-N-(3-hydroxypropyl)aniline, 4-amino-3-methyl-N-propyl-N-(3-hydroxypropyl)aniline, 4-amino-3-propyl-N-methyl-N-(3-hydroxypropyl)aniline, 4-amino-3-methyl-N-methyl-N-(4-hydroxybutyl)aniline, 4-amino-3-methyl-N-ethyl-N-(4-hydroxybutyl)aniline, 4-amino-3-methyl-N-propyl-N-(4-hydroxybutyl)aniline, 4-amino-3-ethyl-N-ethyl-N-(3-hydroxy-2-methylpropyl)aniline, 4-amino-3-methyl-N,N-bis(4-hydroxybutyl)aniline, 4-amino-3-methyl-N,N-bis(5-hydroxypentyl)aniline, 4-amino-3-methyl-N-(5-hydroxypentyl)-N-(4-hydroxybutyl)aniline, 4-amino-3-methoxy-N-ethyl-N-(4-hydroxybutyl)aniline, 4-amino-3-ethoxy-N,N-bis (5-hydroxypentyl)aniline, and 4-amino-3-propyl-N-(4-hydroxybutyl)aniline, and their sulfates, hydrochlorides or p-toluenesulfonates. Among these, particularly, 3-methyl-4-amino-N-ethyl-N-β-hydroxyethylaniline, 4-amino-3-methyl-N-ethyl-N-(3-hydroxypropyl)aniline, and 4-amino-3-methyl-N-ethyl-N-(4-hydroxybutyl)aniline, and their hydrochlorides, p-toluenesulfonates or sulfates are preferred. According to the purpose, two or more of these compounds can be used in combination.

Those described in EP-A-410 450, JP-A-4-11255, can also be used preferably.

Further, these p-phenylenediamine derivatives and sulfates, hydrochlorides, sulfites, and salts of naphthalenedisulfonic acid or p-toluenesulfonic acid may also be used. The amount of the aromatic primary amine developing agent to be used is preferably 0.0002 to 0.2 mol, and more preferably 0.001 to 0.1 mol, per liter of the color developer.

The processing temperature of the color developer in the present invention is generally 20 to 55 °C, and preferably 30 to 55 °C. The processing time for photographing light-sensitive materials is generally 20 sec to 5 min, preferably 30 sec to 3 min and 20 sec, and more preferably 1 min to 2 min and 30 sec. The processing time for printing light-sensitive materials is generally 10 sec to 1 min and 20 sec, preferably 10 sec to 60 sec, and more preferably 10 sec to 40 sec.

Further, if necessary, to the color developer, can be added a sulfite, such as sodium sulfite, potassium sulfite, sodium bisulfite, potassium bisulfite, sodium metasulfite, and potassium metasulfite, or a carbonyl sulfite adduct, as a preservative.

Further, preferably, the above aromatic primary amine color-developing agent is directly added (as the compound for preservation, various hydroxylamines, for example, compounds described in JP-A-63-5341 and JP-A-63-106655, and particularly those having a sulfo group or a carboxyl group, are preferred), hydroxamic acids described in JP-A-63-43138, hydrazides and hydrazines described in JP-A-63-146041, phenols described in JP-A-63-44657 and JP-A-63-58443, α-aminoketones and α-hydroxyketones described in JP-A-63-44656, and/or various saccharides described in JP-A-63-36244. In combination with the above compounds, preferably monoamines described, for example, in JP-A-63-4235, JP-A-63-24254, JP-A-63-21647, JP-A-63-146040, JP-A-63-27841, and JP-A-63-25645; diamines described, for example, in JP-A-63-30845, JP-A-63-14640, and JP-A-63-43139; polyamines described, for example, in JP-A-63-21647, JP-A-63-26655 and JP-A-63-44655; nitroxy radicals described in JP-A-63-53551, alcohols described in JP-A-63-43140 and JP-A-63-53549, oximes described in JP-A-63-56654, or tertiary amines described in JP-A-63-239447, are used.

Further, other preservatives that may be contained, if required, include, for example, various metals described in JP-A-57-44148 and 57-53749, salicylic acids described in JP-A-59-180588, alkanolamines described in JP-A-54-3582, polyethyleneimines described in JP-A-56-94349, and aromatic polyhydroxy compounds described in US-A-3 746 544. In particular, preferably contained are aromatic polyhydroxy compounds. These preservatives are preferably used in an amount of generally 0.005 mol to 0.2 mol, and preferably 0.01 mol to 0.05 mol, per liter of the color developer.

The color-developer for use in the present invention preferably has a pH of generally 9 to 12, and preferably 9.5 to 11.5. To the color developer, other compounds known as ingredients in a developing solution can be contained. To retain the above pH, it is preferable to use various buffers.

Specific examples of the buffer include sodium carbonate, potassium carbonate, sodium bicarbonate, potassium bicarbonate, trisodium phosphate, tripotassium phosphate, disodium phosphate, dipotassium phosphate, sodium borate, potassium borate, sodium tetraborate (borax), potassium tetraborate, sodium o-hydroxybenzoate (sodium salicylate), potassium o-hydroxybenzoate, sodium 5-sulfo-2-hydroxybenzoate (sodium 5-sulfosalicylate), and potassium 5-sulfo-2-hydroxybenzoate (potassium 5-sulfosalicylate). However, buffers for use in the present invention is not limited to these compounds. The amount of the buffer to be added to the color developer is preferably 0.1 mol/liter or more, and particularly preferably 0.1 to 0.4 mol/liter.

In the present invention, various chelating agents can be used in combination within the amount's range not to deteriorate the effects of the compound of the present invention.

As chelating agent, organic acid compounds are preferable, and examples include aminopolycarboxilic acids, organic phosphonic acids, phosphonocarboxylic acids. Specific examples include nitrilotriacetic acid, diethylenetriaminepentaacetic acid, ethylenediaminetetraacetic acid, N,N,N-trimethylenephosphonic acid, ethylenediamine-N,N, N',N'-tetramethylenephosphonic acid, transcyclohexanediaminetetraacetic acid, 1,2-diaminopropanetetraacetic acid, hydrozyethyliminodiacetic acid, glycol ether diaminetetraacetic acid, ethylenediamine bisorthohydroxyphenylacetic acid, 2-phosphonobutane-1,2,4-tricarboxylic acid, 1-hydroxyethylidene-1,1-diphosphonic acid, and N,N'-bis(2-hydroxybenzyl)ethylenediamine-N,N'-diacetic acid. These chelating agents can be additionally used, for example, in an amount of 0.0001 mol to 0.005 mol, per liter of the processing solution.

In the color developer, if necessary, an arbitrary development accelerator can be added.

Examples of the development accelerator include the following compounds: thioether compounds described, for example, in JP-B-37-16088, 3-5987, 38-7826, 44-12380, and 45-9019, and US-A-3 813 247; p-phenylenediamine compounds described in JP-A-52-49829 and 50-15554; quaternary ammonium salts described, for example, in JP-A-50-137726, JP-B-44-30074, and JP-A-56-156826 and 52-43429; amine compounds described, for example, in US-A-2 494 903, 3 128 182, 4 230 796, and 3 253 919, JP-B-41-11431, and US-A-2 482 546, 2 496 926, and 3 582 346; polyalkylene oxides described, for example, in JP-B-37-16088 and 42-25201, US-A-3 128 183, JP-B-41-11431, 42-23883, and US-A-3 532 501; and imidazoles, such as 2-methylimidazole and imidazole .

In addition, it is preferable to add 1-phenyl-3-pyrazolidons as an auxiliary developing agent, to carry out rapid processing.

In the color developer, if required, an arbitrary antifoggant can be added. As the antifoggant, alkali metal halide, such as sodium chloride, potassium bromide, and potassium iodide, and organic antifoggant, can be used. Typical examples of the organic antifoggant include nitrogen-containing heterocyclic compounds, such as, benzotriazole, 6-nitrobenzimidazole, 5-nitoroisoindazole, 5-methylbenzotriazole, 5-nitrobenzotriazole, 5-chloro-benzotriazole, 2-thiozolyl-benzimidazole, 2-thiazolylmethyl-benzimidazole, indazole, hydroxyazaindolizine, adenine.

Further, the color developer may contain a fluorescent whitening agent. As the fluorescent whitening agent, 4,4-diamino-2,2'-disulfostilbene-series compounds are preferable. The amount to be added is generally 0 to 5 g/liter, and preferably 0.1 to 4 g/liter.

Further, if necessary, various surface-active agents, such as alkylsulfonic acids, arylsulfonic acids, aliphatic carboxylic acids, and aromatic carboxylic acids, may be added.

The black-and-white first developing solution used for color reversal processing, and the black-and-white developing solution for black-and-white silver halide light-sensitive materials, for which the compound of the present invention can be used, can contain well-known various additives generally added.

Representative additives include developing agents, such as 1-phenyl-3-pyrazolidone, Metol (trade name), and hydroquinone; preservatives, such as sulfites; accelerators comprising alkalis, such as sodium hydroxide, sodium carbonate, and potassium carbonate; organic or inorganic inhibitors, such as potassium bromide, 2-methylbenzimidazole, and methylbenzthiazole; water softeners, such as polyphosphates; and development inhibitors comprising, for example, a trace amount of iodides and mercapto compounds.

The bleaching solution in which the compound of the present invention can be used contains at least an oxidizing agent for oxidizing silver and a rehalogenating agent (or an organic ligand instead). As the bleaching agent, for example, known polyaminocarboxylic acid iron(III) complex salts, hydrogen peroxide, persulfates, or hydrobromides can be used, and they may be used in combination. The amount of the bleaching agent to be used is generally 0.05 to 2 mol, and preferably 0.1 to 5 mol, per liter of the bleaching solution. As the rehalogenating agent, generally a halide, such as a chloride, a bromide, or an iodide, is used, but instead an organic ligand capable of forming a slightly soluble silver salt can be used. The amount thereof to be used is generally 0.1 to 2 mol/liter, and preferably 0.3 to 1.5 mol/liter.

The above halide is added in. the form of an alkali metal salt, an ammonium salt, a guanidine salt, an amine salt. Specific examples include sodium bromide, ammonium bromide, potassium chloride, and guanidine hydrochloride, with preference given to ammonium bromide.

By adding the compound of the present invention to the bleaching solution, the preservability of the bleaching solution can be improved, and this is particularly remarkable when hydrogen peroxide, a persulfate, or a hydrobromide is used as the bleaching agent.

The bleach-fix solution to which the compound of the present invention can be added contains, in addition to the bleaching agent, the below-described fixing agent, and if necessary the above rehalogenating agent. The amount of the bleaching agent in the bleach-fix solution to be used is the same as that of the bleaching solution. The amount to be used of the rehalogenating agent is generally 0 to 2.0 mol/liter, and preferably 0.01 to 1.0 mol/liter.

By adding the compound of the present invention to the bleach-fix solution, the preservability of the solution is improved.

Further, to the bleaching solution or the bleach-fix solution according to the present invention, are added a bleach accelerator, a corrosion inhibitor, for preventing a processing bath tank from being corroded, a buffer, for keeping the pH of the solution, a fluorescent whitening agent, an antifoaming agent as required. Preferable conditions of the bleaching solution, the bleach-fix solution, and the fixing solution in the present invention are the same as those of the processing solution that may contain the metal chelate compound of the present invention.

In the fixing solution according to the present invention, by adding the compound represented by formula (I), the preservability of the solution is improved; as well as that the iron ions carried in from the bleaching solution can be sequestered, to improve the preservability of the solution.

The same effects can be obtained by adding the compound of the present invention to washing water and/or a stabilizing solution.

The washing water used in the washing step and the stabilizing solution may contain various surface-active agents, to prevent the processed light-sensitive material from being dried unevenly due to water droplets. Examples of the surface-active agents include polyethylene glycol-type nonionic surface-active agents, polyhydric alcohol-type nonionic surface-active agents, alkylbenzenesulfonate-type anionic surface-active agents, higher alcohol sulfate-type anionic surface-active agents, alkylnaphthalenesulfonate-type anionic surface-active agents, quaternary ammonium

salt-type cationic surface-active agents, amine salt-type cationic surface-active agents, amino acid-type amphoteric surface-active agents, and betaine-type amphoteric surface-active agents. Among these, since ionic surface-active agents sometimes combine with various ions introduced unintentionally along with the processing to form insoluble substances, nonionic surface-active agents are preferably used, with particular preference given to alkylphenol ethylene oxide adducts. The alkylphenol is particularly preferably octylphenol, nonylphenol, dodecylphenol, or dinonylphenol, and the addition molar number of ethylene oxide is particularly preferably 8 to 14 (mol). Further, a silicon-series surface-active agent high in antifoaming effect is also preferably used.

Further, the washing water or the stabilizing solution can contain a variety of bacteria-proofing agents and mildew-proofing agents, to prevent the formation of scale or the existence of mildew occurring on processed light-sensitive materials. As the bacteria-proofing agents and mildew-proofing agents, can be used, for example, one or more of thiazorilbenzimidazole compounds, as described in JP-A-57-157244 and JP-A-58-105145, isothiazolone compounds, as described in JP-A-54-27424 and JP-A-57-8542, and general-purpose mildew-proofing agents described in "Journal Antibacteria and Antifungus Agents," Vol. 1, No. 5, pp 207 to 223 (1983), such as chlorophenol compounds, represented by trichlorophenol; bromophenol compounds, organotin compounds, organozinc compounds, thiocyanic acid compounds, isothiocyanic acid compounds, acid amide compounds, diazine compounds, triazine compounds, thiourea compounds, benzotriazolealkylguanidine compounds, quaternary ammonium salts, represented by benzalkonium chloride; or antibiotics, such as penicillins. Further, various fungicides described in JP-A-48-83820 can be used.

Further, preferably, various chelating agents are additionally used, in the range in which the effects of the compound of the present invention is not impaired. Preferable compounds as the chelating agents include, for example, aminopolycarboxylic acids, such as ethylenediaminetetraacetic acid and diethylenetriaminepentaacetic acid, organic phosphonic acids, such as 1-hydroxyethylidene-1,1-diphosphonic acid and ethylenediamine-N,N,N',N'-tetramethylenephosphonic acid, and hydrolyzates of maleic anhydride polymers described in EP-A-345 172 (A1).

Further, the above preservatives that can be contained in the fixing solution or the bleach-fix solution are preferably contained in the washing water.

The stabilizing solution used can be, for example, an organic acid, a solution having a buffering action with a pH of 3 to 6, or a solution containing an aldehyde (e.g. formalin and glutaraldehyde), hexamethylenetetramine, hexahydrotriazine, or an N-methylol compound (e.g. dimethylolurea and N-methylolpyrazole), and, in addition thereto, if necessary, for example, an ammonium compound, such as ammonium chloride and ammonium sulfite, a compound of a metal, such as Bi and Al; a fluorescent whitening agent, a hardener, and an alkanolamine described in US-A-4 786 583 can be used.

Further, the washing step and the stabilizing step are preferably of a multi-stage counter current system, and the number of stages is preferably 2 to 4. The replenishment rate is generally 2 to 30 times, and preferably 2 to 15 times, the amount carried in from the preceding bath per unit area.

As the water used in the washing step or the stabilizing step, besides tap water, water deionized with ion exchange resins to bring the concentrations of Ca and Mg to 5 mg/liter or below, or water sterilized with a halogen lamp, an ultraviolet germicidal lamp is preferably used.

As the water to supplement the evaporation, tap water can be used, but deionized water or sterilized water preferably used in the washing step or the stabilizing step, is preferably used.

In each of the processing solutions according to the present invention, preferably, stirring of the processing solution is intensified as much as possible. Methods for intensifying stirring that can be mentioned include a method described in JP-A-62-183460, wherein a jet stream of a processing solution is struck against the emulsion surface of a light-sensitive material; a method described in JP-A-62-18346, wherein the stirring effect is increased using a rotating means; a method wherein a light-sensitive material is moved with the emulsion surface thereof in contact with a wiper blade or squeeze rollers provided in a solution, to cause the solution near the emulsion surface to become turbulent, to improve the stirring effect; and a method wherein the overall circulated flow rate of a processing solution is increased.

The processing method of the present invention is preferably carried out using an automatic processor. The conveying method in the automatic processor is described in JP-A-60-191257, JP-A-60-191258, and JP-A-60-191259. To carry out rapid processing using the processing composition of the present invention, in an automatic processor, it is preferable to shorten the crossover between the processing tanks. An automatic processor wherein the crossover time is 10 sec or less is described in JP-A-1-319038.

When continuous processing is carried out by using an automatic processor by the processing method of the present invention, to compensate for the consumption of components of processing solutions in the course of the processing of the light-sensitive material, and to suppress the accumulation of undesired. components dissolved out from the light-sensitive material into the processing solutions, replenishers are preferably added, in accordance with the amount of the processed light-sensitive material. Each of the processing steps may be provided with two or more processing tanks, and in that case the replenisher is preferably supplied in a countercurrent manner, wherein the replenisher is flowed from the succeeding tank to the preceding tank. Particularly, in the water washing step and the stabilizing step, a 2-stage to 4-stage cascade is preferably used.

The replenishing rate is preferably reduced as much as possible, except under the circumstances that the change in the composition of each processing solution causes disadvantageous photographic performance or disadvantageous solution contamination.

The amount of the color-development replenisher is, in the case of a color photographing light-sensitive material, generally 100 to 1,500 ml, and preferably 100 to 1,000 ml, per $m^2$ of the light-sensitive material, and, in the case of a color print light-sensitive material, generally 20 to 220 ml, and preferably 30 to 160 ml, per $m^2$ of the light-sensitive material.

The amount of the bleach replenisher is, in the case of a color photographing light-sensitive material, generally 10 to 500 ml, and preferably 10 to 160 ml, per $m^2$ of the light-sensitive material, and, in the case of a color print light-sensitive material, generally 20 to 300 ml, and preferably 50 to 150 ml, per $m^2$ of the light-sensitive material.

The amount of the bleach-fix replenisher is, in the case of a color photographing light-sensitive material, generally 100 to 3,000 ml, and preferably 200 to 1,300 ml, per $m^2$ of the light-sensitive material, and, in the case of a color print light-sensitive material, generally 20 to 300 ml, and preferably 50 to 200 ml, per $m^2$ of the light-sensitive material. The replenishment of the bleach-fix solution may be carried out in the form of one solution, or in the separate form of a bleaching composition and a fixing composition, or the bleach-fix replenisher may be made by mixing the overflow of the bleaching bath and/or the fixing bath.

The amount of the fixing replenisher is, in the case of a color photographing light-sensitive material, generally 300 to 3,000 ml, and preferably 300 to 1,000 ml, per $m^2$ of the light-sensitive material, and, in the case of a color print light-sensitive material, generally 20 to 300 ml, and preferably 50 to 200 ml, per $m^2$ of the light-sensitive material.

The replenishment rate of the washing water or the stabilizing solution is generally 1 to 30 times, and preferably 2 to 15 times, the amount carried in from the preceding bath per unit area.

So as to reduce the amount of the replenisher further for the preservation of the environment, preferably different regeneration (reclaiming) methods are used in combination. The regeneration may be carried out by circulating the processing solution in an automatic processor; or by removing the processing solution from the processing tank once, then subjecting the processing solution to a suitable regeneration process, and returning the thus processed processing solution into the processing tank as a replenisher.

The regeneration of the developer can be carried out by ion exchange using an anion exchange resin, electrodialysis, to remove accumulated matter, and/or addition of a chemical called a regenerant. The regeneration rate is preferably 50% or more, and more preferably 70% or more. As the anion exchange resin, commercially available ones can be used, but a highly selective ion exchanger described in JP-A-63-11005 is preferably used.

Photographic light-sensitive materials that can be processed with the processing composition of the present invention and that can be mentioned include, for example, usual black-and-white silver halide photographic light-sensitive materials (e.g. photographing black-and-white light-sensitive materials, X-ray black-and-white light-sensitive materials, and printing black-and-white materials), usual multi-layer silver halide color photographic light-sensitive materials (e.g. color negative films, color reversal films, color positive films, movie color negative films, color print papers, reversal color print papers, and direct positive color print papers), infrared light-sensitive materials for laser scanners, and diffusion transfer light-sensitive materials (e.g. silver diffusion transfer light-sensitive materials and color diffusion transfer light-sensitive materials). The photographic light-sensitive materials according to the present invention may carry a magnetic record.

As the light-sensitive material used in the present invention, the following can be used preferably: a light-sensitive material having a magnetic recording layer, which recording layer comprises magnetic particles (preferably made of Co-covered ferromagnetic iron oxide) that are dispersed in a binder; the recording layer is preferably optically transparent and is provided all over the surface of the light-sensitive material. The magnetic particles may be treated with a coupling agent, as described in JP-A-6-161032. As the binder, a polymer described, for example, in JP-A-4-219569 can be preferably used. Although the recording layer may be provided anywhere, it is preferably provided on the side (backing layer) of the base opposite to the emulsion layer. Preferably, for example, a layer containing a slip agent (lubricant) is provided on the recording layer, and the outermost layer on the base on the side of the light-sensitive emulsion layer contains a matting agent.

Further, preferably, the light-sensitive material contains an antistatic agent, so as to provide the light-sensitive material with antistatic properties, even after the development processing. As the antistatic agent, an electrically conductive metal oxide and an ionic polymer are preferable. The antistatic agent is preferably used in such a way that the electrical resistance may be $10^{12}$ Ω.cm or less under conditions of 25 degrees (°C) and 10% RH.

Light-sensitive materials having a magnetic recording layer are described in US-A-5 336 589, US-A-5 250 404, US-A-5 229 259, US-A-5 215 874, and EP-A-466 130 (A).

Further, as the base used for the light-sensitive material, a polyester base that is improved with respect to core-set curl and that is made thin, is preferable. Preferably, the thickness is 50 to 105 µm, and the polyester is a polyethylene aromatic dicarboxylate-series polyester (preferably, mainly made of benzenedicarboxylic acid, naphthalenedicarboxylic acid, and ethylene glycol). Preferably the glass transition temperature is 50 to 200 degrees. Further,

as the surface treatment of the base, ultraviolet-light irradiation treatment, corona discharge treatment, glow discharge treatment, and flame treatment are preferable. Further, before or after the application of an undercoat layer (a subbing layer) to the base but before the application of emulsion layers, preferably the base is heat-treated at a temperature ranging from 40 degrees to the glass transition temperature of the base, for 0.1 to 1500 hours. In addition to bases, light-sensitive materials, development processing, cartridges (magazines), etc., are described in Kokai-Giho, Kogi No. 94-6023 (published by Hatsumei-Kyokai, 1994).

The photographic light-sensitive material according to the present invention can have various layer structures (having, for example, silver halide emulsion layers photosensitive to red, green, and blue, respectively, an undercoat layer, an antihalation layer, a filter layer, an intermediate layer, and a surface protective layer) that may be arranged in various ways on one surface or both surfaces to meet the purpose of the light-sensitive material.

In the color light-sensitive material that can be applied to the process of the present invention, various color couplers can be used, and specific examples thereof are described in patents set forth in the above-mentioned RD No. 17643, VII-C-G, RD No. 307105, VII-C-G, JP-A-62-215272, JP-A-3-33847, JP-A-2-33144, EP-A-447 969 (A), and EP-A-482 552 (A).

As the yellow couplers, those described, for example, in US-A-3 933 501, 4 022 620, 4 326 024, 4 401 752, and 4 248 961, JP-B-58-10739, British Patent Nos. 1 425 020 and 1 476 760, US-A-3 973 968, 4 314 023, and 4 511 649, 5 118 599, EP-A-249 473, and 0 447 969, and JP-A-63-23145, 63-123047, 1-250944, 1-213648, can be used alone or in combination as long as not inhibit the effects of the present invention.

Particularly preferable yellow couplers include yellow couplers represented by formula (Y), as described in JP-A-2-139544, page 18, upper left column, to page 22, lower left column; acylacetamide-series yellow couplers characterized by an acyl group, as described in JP-A-5-2248 and EP-A-0 447 969; and yellow couplers represented by formula (Cp-2), as described in JP-A-5-27389 and EP-A-0 446 863 (A2).

As the magenta couplers, 5-pyrazolone-series compounds and pyrazoloazole-series compounds are preferable, and those described, for example, in US-A-4 310 619 and 4 351 897, EP-A-73 636, US-A-3 061 432 and 3 725 067, Research Disclosure No. 24220 (June, 1984), JP-A-63-33552, Research Disclosure No. 24230 (June, 1984), JP-A-60-43659, 61-72238, 60-35730, 55-118034, and 60-185951, US-A-4 500 630, 4 540 654, and 4 556 630, and International Publication No. WO 88/04795, are more preferable.

Particularly preferable magenta couplers include pyrazoloazole-series magenta couplers of formula (I), as described in JP-A-2-139544, page 3, lower right column, to page 10, lower right column; and 5-pyrazolone magenta couplers of formula (M-1), as described in JP-A-2-139544, page 17, lower left column, to page 21, upper left column. The above pyrazoloazole-series magenta couplers are most preferable.

As the cyan couplers, phenol-series couplers and naphthole-series couplers can be mentioned, and those described, for example, in US-A-4 052 212, 4 146 396, 4 228 233, 4 296 200, 2 369 929, 2 801 171, 2 772 162, 2 895 826, 3 772 002, 3 758 308, 4 334 011, and 4 327 173, West German Patent Publication No. 3329729, EP-A-0 121 365 and 0 249 453, US-A-3 446 622, 4 333 999, 4 775 616, 4 451 559, 4 427 767, 4 690 889, 4 254 212, and 4 296 199 and JP-A-61-42658 are preferable. In addition, pyrazoloazole-series couplers described in JP-A-64-553, 64-554, 64-555, and 64-556; pyrrolotriazole-series couplers described in EP-A-0 488 248 and 0 491 197; pyrroloimidazole-series couplers described in EP-A-0 456 226; pyrazolopyrimidine-series couplers described in JP-A-64-46753; imidazole-series couplers described in US-A-4 818 672 and JP-A-2-33144; cyclic active-methylene-type cyan couplers described in JP-A-64-32260; or couplers described in JP-A-1-183658, 2-262655, 2-85851 and 3-48243, can also be used.

Typical examples of the polymerized dye-forming couplers include those described, for example, in US-A-3 451 820, US-A-4 080 211, US-A-4 367 282, US-A-4 409 320, US-A-4 576 910, GB-A-2 102 137, and EP-A-341 188 (A).

The couplers whose color-formed dyes are suitably diffusible are preferably those described in US-A-4 366 237, GB-A-2 125 570, EP-A-96 570, and West German Patent (OLS) 3 234 533.

Compounds that can release a photographically useful residual group as a result of coupling can also be used in the present invention. As DIR couplers that release a development inhibitor, those described in patents described in the above-mentioned Research Disclosure No. 17643, VII-F, as well as JP-A-57-151944, 57-154234, 60-184248, and 63-37346, and US-A-4 248 962 and 4 782 012, are preferable.

As couplers that release nucleus-forming agents (nucleators) or development accelerators image-wise at the time of development, those described in GB-2 097 140 and 2 131 188, and JP-A-59-157638 and 59-170840, are preferable.

Further examples of couplers that can be used in the color photographic element according to the present invention include for example, competing couplers described in US-A-4 130 427, multi-equivalent couplers described in US-A-4 283 472, 4 338 393, and 4 310 618; DIR redox-compound-releasing couplers, DIR coupler-releasing couplers, DIR coupler-releasing redox compounds, or DIR redox-releasing redox compounds, described in JP-A-60-185950 and 62-24252; couplers capable of releasing color-restorable dyes after split-off, as described in EP-A-173 302, bleach accelerator-releasing couplers described in Research Disclosure Nos. 11449 and 24241 and JP-A-61-201247, ligand-releasing couplers described in US-A-4 553 477, couplers capable of releasing leuco dyes, as described in JP-A-63-75747, and couplers capable of releasing fluorescent dyes, as described in US-A-4 774 181.

Appropriate support for use in the present invention is described, for example, in the above-mentioned Research Disclosure (RD) No. 17643, page 28 and ibid. No. 18716, page 647, right column to page 648, left column.

Particularly, the base used for color negative films is preferably one having a conductive layer and a transparent magnet layer on one surface, as described in JP-A-4-62543, one having a magnetic recording layer, as described in International Publication WO-A-90/04205, FIG. 1A, or one having a striped magnetic recording layer and a transparent magnetic recording layer adjacent to the striped magnetic recording layer, as described in JP-A-4-124628. Preferably, on these magnetic recording layers, is provided a protective layer described in JP-A-4-73737.

Preferably the thickness of the base is 70 to 120 μm, and as the material of the base, various plastic films described in JP-A-4-124636, page 5, upper right column, line 1, to page 6, upper right column, line 5, can be used. Preferable ones include cellulose derivatives (e.g. diacetyl acetate, triacetyl acetate, propionyl acetate, butanoyl acetate, and acetylpropionyl acetate) and polyesters, as described in JP-B-48-40414 (e.g. polyethylene terephthalates, poly-1,4-cyclohexanedimethylene terephthalates, and polyethylene naphthalates). Preferably the film base used in the present invention is a polyester base, since it is very high in draining effect.

Further, the package (patrone, cartridge) for housing the color negative film of the present invention may be any of currently used ones or known ones, and particularly preferably it is one having the shape described in US-A-4 834 306, FIG. 1 to FIG. 3, or one described in US-A-4 846 418, FIG. 1 to FIG. 3.

Furthermore, the color negative film used in the present invention is preferably one having the subject matter described in JP-A-4-125558, page 14, upper left column, line 1, to page 18, lower left column, line 11.

According to the present invention, the oxidation and decomposition of components of processing solutions by the action of metal ions can be suppressed in a wide pH range. Further, processing in a wide pH range can be performed stably with respect to desilvering and photographic properties, and it can be performed using a processing agent that pollutes the environment little.

The present invention will be described in more detail with reference to examples, but the present invention is not restricted to them.

## EXAMPLE

### Example 1

Sample 101, which was a photographing multi-layer color light-sensitive material (color negative film) described in Example 1 of JP-A-5-303186, was prepared.

Sample 101 was cut to a width of 35 mm, it was exposed imagewise, and it was continuously processed with the below-shown processing solutions, until the replenishing rate of the bleach-fix solution reached 10 times the tank volume.

The bleach-fix solution was passed through a silver recovering apparatus in an in-line manner, to recover silver. Part of the overflow from the silver recovering apparatus was discharged as waste liquor, and the remainder was regenerated and was reused as a replenisher of the bleach-fix solution. The silver recovering apparatus was a small-sized electrolytic silver recovering apparatus, wherein the anode was made of carbon and the cathode was made of stainless steel, and the current density used was 0.5 $A/dm^2$. A schematic view of the system for recovering silver is illustrated in Fig. 1 of JP-A-6-175305. That is, an overflow 21 of a bleach-fix tank 20 was connected directly to the silver recovering apparatus 22, and part of the overflow, in an amount of 100 ml per 1 min, was returned to the original bleach-fix tank 20, by a pump 23 through a filter 24. An overflow 25 from the silver recovering apparatus 22 was recovered, in an amount of 300 ml per liter of the overflow, to a regeneration tank 26. When the recovered amount reached 1 liter, air was bubbled there-through for about 2 hours; then a regenerator 28 was added, and the resulting solution was sent to a replenishing tank 30 of the bleach-fix solution by a pump 29. The remainder (100 ml) was discharged at 27 as waste liquor. The amount of the waste liquor was 220 ml per $m^2$ processing of Sample 101.

The washing process was carried out using 5-stage multi-chamber water washing tanks arranged side by side in a countercurrent cascade manner. Specifically, use was made of one shown in Fig. 1 of JP-A-5-66540.

The overflow of first washing water $W_1$ was cascaded to the previous bleach-fix tank. A reverse osmosis membrane (RO) apparatus RC 30 (manufactured by Fuji Photo Film Co., Ltd.) was placed between the fourth washing $W_4$ and the fifth washing $W_5$. Namely, washing water taken from $W_4$ was passed through the RO apparatus, the condensed solution was returned to $W_4$, and the passed solution was returned to $W_4$. A schematic diagram of the processor is shown in Fig. 2 of JP-A-6-175305. The processing step ($N_{Blix}$) is shown below.

| Processing step ($N_{Blix}$) | | | | |
|---|---|---|---|---|
| Processing step | Processing | | Replenishing rate*1 | Tank Volume/liter |
| | time | temperature | | |
| Color developing | 1 min 50 sec | 45 °C | 104 ml | 2 |
| Bleach-Fixing | 1 min 50 sec | 45 °C | 200 ml | 2 |
| Washing (1) | 15 sec | 45 °C | - | 0.5 |
| Washing (2) | 15 sec | 45 °C | - | 0.5 |
| Washing (3) | 15 sec | 45 °C | - | 0.5 |
| Washing (4) | 15 sec | 45 °C | - | 0.5 |
| Washing (5) | 15 sec | 45 °C | 104 ml | 0.5 |
| Stabilizing | 2 sec | Room temperature | 30 ml | Coated |
| Drying | 50 sec | 70 °C | - | - |

*1 the replenishment rate was the amount per 1 $m^2$ of the light-sensitive material

The crossover time from the color development to the bleach fix, and from the bleach-fix to the washing (1), was 3 sec. This crossover time was included in the processing time in the preceding bath. Moreover, the average amount carried over per $m^2$ of the light-sensitive material was 65 ml.

The temperature and relative humidity of the atmosphere around each processor were detected by a hygrothermal meter, as shown in JP-A-3-280042, to calculate the evaporated amount, and the evaporation was compensated for each tank. As the water for the compensation, deionized water for the above washing water was used.

The composition of the processing solutions is shown below:

| (Color-developer) | Mother Solution (g) | Replenisher (g) |
|---|---|---|
| Diethylenetriaminepentaacetic acid | 1.2 | 4.0 |
| 1-Hydroxyethylidene-1,1-diphosphonic acid | 2.7 | 3.3 |
| Potassium hydroxide | 2.50 | 3.90 |
| Sodium sulfite | 3.84 | 9.0 |
| Sodium bicarbonate | 1.8 | - |
| Potassium carbonate | 31.7 | 39.0 |
| Potassium bromide | 5.60 | - |
| Potassium iodide | 1.3 mg | - |
| Hydroxylamine sulfate | 2.5 | 6.9 |
| 2-Methyl-4-[N-ethyl-N-(β-hydroxyethyl)amino]aniline sulfate | 9.0 | 18.5 |
| Water to make | 1.0 liter | 1.0 liter |
| pH | 10.05 | 11.90 |

| (Bleach-Fixing solution) | Mother solution (mol) | Replenisher at start (mol) |
|---|---|---|
| Ammonium thiosulfate | 1.4 | 2.31 |
| Chelating agent described in Table | 1 0.17 | 0.28 |
| Ferric nitrate nonahydrate | 0.15 | 0.25 |
| Ammonium bisulfate | 0.10 | 0.17 |
| m-Carboxybenzenesulfinic acid | 0.05 | 0.09 |
| Water to make | 1.0 liter | 1.0 liter |
| pH (25 °C) | 6.0 | 6.0 |
| (pH was adjusted by acetic acid and aqueous ammonia) | | |

| (Bleach-Fixing regenerating agent) (Amount added per liter of the recovered solution for regeneration (g)) | |
|---|---|
| Ammonium thiosulfate | 0.91 |
| Chelating agent described in Table 1 | 0.11 |
| Ferric nitrate nonahydrate | 0.10 |
| Ammonium sulfite | 0.07 |
| m-Carboxybenzenesulfinic acid | 0.04 |

(Washing water) (Both mother solution and replenisher)

Tap water was treated by passage through a mixed bed ion-exchange column filled with H-type strong acidic cation exchange resin (Amberlite IR-120B, trade name, made by Rohm & Haas) and OH-type strong basic anion exchange resin (Amberlite IRA-400, the same as the above) so that the concentrations of Ca ions and Mg ions in water were both made to decrease to 3 mg/liter or below, followed by adding 20 mg/liter of sodium dichlorinated isocyanurate and 150 mg/liter of sodium sulfate. The pH of this water was in the range of 6.5 to 7.5.

| (Stabilizing solution) for coating | (g) |
|---|---|
| Formalin (37%) | 2.0 ml |
| Polyoxyethylene-p-monononylphenylether (av. polymerization degree: 10) | 0.3 |
| Disodium ethylenediaminetetraacetate | 0.05 |
| Water to make | 1.0 liter |
| pH | 5.0 to 8.0 |

The above processing system was carried out, and the amount of the waste liquor was 22 liters after 100 $m^2$ of Sample 101 was processed.

The amount of residual silver in the maximum density section of the multi-layer color light-sensitive Sample 101 subjected to the above processing was measured by fluorescent X-ray analysis. The result is shown in Table 1. The $D_{min}$ values of these samples obtained by processing in this manner were measured using green light (G light) to be read, respectively.

Then, the bleach-fix step of the above processing was changed to four steps, bleach-washing (A)-washing (B)-fixing, as a reference processing method free from bleach fogging, and the processing was carried out using the below-shown processing solution formulation. There was no change, except for the below-shown changes.

| Processing step | Processing | | Replenisher rate |
|---|---|---|---|
| | time | temperature | |
| Bleaching | 3 min 00 sec | 38 °C | 710 ml |
| Washing (A) | 15 sec | 24 °C | Countercurrent piping system from (B) to (A) |
| Washing (B) | 15 sec | 24 °C | 430 ml |
| Stabilizing | 3 min 00 sec | 38 °C | 430 ml |

| (Basic bleaching solution) | Tank solution (g) | Replenisher (g) |
|---|---|---|
| Ethylenediaminetetraacetic acid ferric complex salt sodium trihydrate | 100.0 | 120.0 |
| Disodium ethylenediaminetetraacetate | 10.0 | 11.0 |
| 3-Mercapto-1,2,4-triazole | 0.03 | 0.08 |
| Ammonium bromide | 140.0 | 160.0 |
| Ammonium nitrate | 30.0 | 35.0 |
| Aqueous ammonia (27%) | 6.5 ml | 4.0 ml |
| Water to make | 1.0 liter | 1.0 liter |
| pH | 6.0 | 5.7 |
| (pH was adjusted by aqueous ammonia and nitric acid) | | |

| (Fixing solution) | Tank solution (g) | Replenisher (g) |
|---|---|---|
| Disodium ethylenediaminetetraacetate | 0.5 | 0.7 |
| Ammonium sulfite | 20.0 | 22.0 |
| Aqueous ammonium thiosulfate solution (700 g/liter) | 295.0 ml | 320.0 ml |
| Acetic acid (90%) | 3.3 | 4.0 |
| Water to make | 1.0 liter | 1.0 liter |
| pH | 6.7 | 6.8 |
| (pH was adjusted by aqueous ammonia and acetic acid) | | |

Similarly, the Dmin values of the processed light-sensitive materials obtained by using the above reference bleaching solution were read. The differences, the ΔDmin, between these Dmin values of the light-sensitive materials and the Dmin of the reference bleaching solution as a reference, were found. In passing, the Dmin value obtained by using the reference bleaching solution was 0.60.

Bleach fog (ΔDmin) = (Dmin of each sample) -

(Dmin of the reference bleaching solution)

Then, using the above Sample 101, the increase in stain during storage of the processed light-sensitive material was found, from a change in density of Dmin of the color-unformed section between those before and after storage under the following conditions.
Dark/heat and humidity conditions: 60 °C, 70% RH, 4 weeks

Stain increase (ΔD) = (Dmin after storage) -

(Dmin before storage)

To investigate the degree of insufficiency of color formation, Sample 101 processed in the processing step ($N_{Blix}$) was processed in the below processing step ($N_{BL}$) again.

| Processing step ($N_{BL}$) | | | |
|---|---|---|---|
| Processing step | Processing | | Replenishing rate*1 |
| | time | temperature | |
| Bleaching | 3 min 00 sec | 38 °C | 710 ml |
| Washing (C) | 15 sec | 24 °C | Countercurrent piping system from (D) to (C) |
| Washing (D) | 15 sec | 24 °C | 430 ml |
| Stabilizing | 3 min 00 sec | 38 °C | 430 ml |
| Washing (6) | 15 sec | 45 °C | - |
| Washing (7) | 15 sec | 45 °C | - |
| Washing (8) | 15 sec | 45 °C | - |
| Washing (9) | 15 sec | 45 °C | - |
| Washing (10) | 15 sec | 45 °C | 104 ml |
| Stabilizing | 2 sec | Room temperature | 30 ml |
| Drying | 50 sec | 70 °C | - |

*1 the replenishment rate was the amount per 1 $m^2$ of the light-sensitive material

For the bleaching, washing, fixing, and stabilizing, the above reference bleaching solution, washing water, fixing solution, and stabilizing solution were used, respectively.
The Dmax values of Sample 101, measured with red light (R light) before and after processed in the above processing step ($N_{BL}$), were read.

$$\text{Insufficiency of color formation } (\Delta Dmax) = (Dmax \text{ after processed in } N_{BL}) - (Dmax \text{ of each sample})$$

The chelating agents used in the bleach-fix solution and the bleach-fix regenerator were respectively changed to those shown in Table 1, and tests of Nos. 101 to 110 were carried out.
The results of the above evaluation are shown in Table 1.

Table 1

| No. | Chelating agent | Amount of residual silver $\mu g/cm^2$ | Bleach fogging $\Delta D_{min}$ (G) | Stain increase $\Delta D$ (G) | Insufficiency of color formation $\Delta D_{max}$ (R) | Remarks |
|---|---|---|---|---|---|---|
| 101 | Comparative compound A | 48.5 | 0.00 | 0.01 | 0.00 | Comparative example |
| 102 | 〃 B | 39.7 | 0.08 | 0.08 | 0.00 | 〃 |
| 103 | 〃 C | 25.2 | 0.00 | 0.01 | 0.20 | 〃 |
| 104 | 〃 D | 8.8 | 0.01 | 0.01 | 0.18 | 〃 |
| 105 | Exemplified compound 1 | 5.1 | 0.00 | 0.01 | 0.00 | This invention |
| 106 | 〃 4 | 4.4 | 0.00 | 0.01 | 0.00 | 〃 |
| 107 | 〃 5 | 6.7 | 0.00 | 0.01 | 0.00 | 〃 |
| 108 | 〃 27 | 7.5 | 0.00 | 0.01 | 0.01 | 〃 |
| 109 | 〃 28 | 7.8 | 0.00 | 0.01 | 0.01 | 〃 |
| 110 | Comparative compound 37 | 8.1 | 0.01 | 0.02 | 0.02 | 〃 |

Comparative compound A

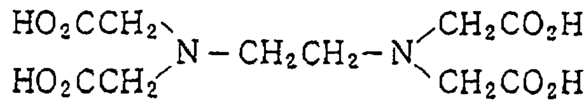

EDTA

Comparative compound B

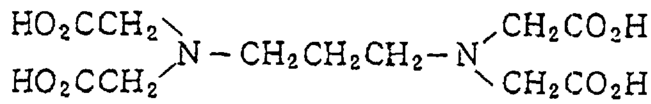

Comparative compound C

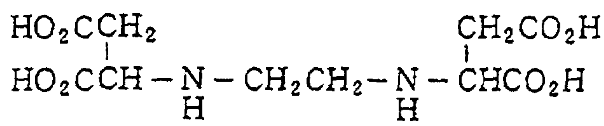

EDDS

Comparative compound D

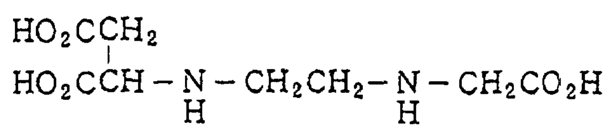

EDMS

As is apparent from the results shown in Table 1, the superiority of the present invention, which takes desilvering properties, bleach fogging, stain increase, and insufficiency of color formation comprehensively and satisfactorily into account, is apparent.

Example 2

A multi-layered color photographic printing paper (sample 001) according to Example 4 in JP-A-5-303186 and the following processing solutions were prepared.

| (Color-developer) | Tank Solution | Replenisher |
|---|---|---|
| Cation-exchanged water | 800.0ml | 800.0 ml |
| Compound M | 0.1 g | 0.1 g |

Compound M

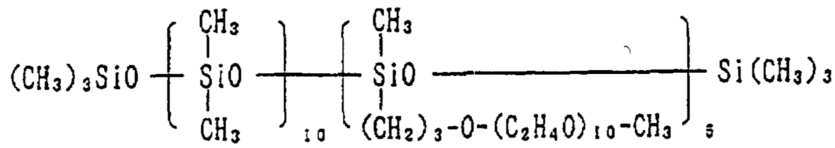

| | | |
|---|---|---|
| Triisopropanolamine | 15.0 g | 15.0 g |
| Potassium hydroxide | 3.0 g | 3.0 g |
| Ethylenediaminetetraacetic acid | 4.0 g | 4.0 g |
| Sodium 4,5-dihydroxybenzene | | |

| | | |
|---|---|---|
| -1,3-disulfonate | 0.5 g | 0.5 g |
| Potassium chloride | 14.5 g | - |
| Potassium bromide | 0.04 g | - |
| Fluorescent whitening agent | | |
| (Compound N) | 2.5 g | 3.0 g |

Compound N

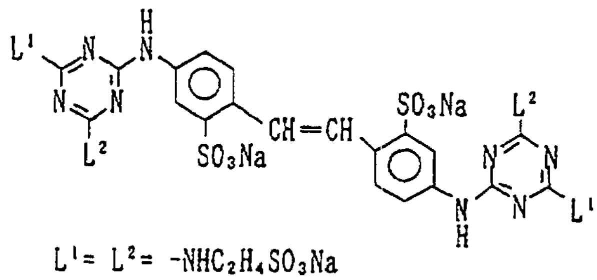

$L^1 = L^2 = -NHC_2H_4SO_3Na$

| | | |
|---|---|---|
| Sodium sulfite | 0.1 g | 0.1 g |
| Disodium-N,N-bis(sulfonatoethyl)hydroxylamine | 8.5 g | 11.0 g |
| N-Ethyl-N-(β-methanesulfonamidoethyl)-3-methyl-4-aminoaniline •3/2 sulfuric acid•monohydrate | 5.0 g | 11.5 g |
| Potassium carbonate | 26.3 g | 26.3 g |
| Water to make | 1000.0 ml | 1000.0 ml |
| pH | 10.15 | 11.15 |

(at 25 °C / pH was adjusted by KOH or sulfuric acid)

(Bleach-Fixing solution)

| | | |
|---|---|---|
| Water | 700.0 ml | 700.0 ml |
| Ammonium thiosulfate (750 g/liter) | 100.0 ml | 250.0 ml |
| Ammonium sulfite | 35.0 g | 88.0 g |
| p-Aminobenzensulfinic acid | 5.0 g | 12.5 g |
| Imidazole | 8.0 g | 20.0 g |
| Chelating agent (shown in Table 2) | 0.11 mol | 0.28 mol |
| Ferric nitrate nonahydrate | 40.4 g | 101 g |
| Water to make | 1000.0 ml | 1000.0 ml |
| pH | 7.0 | 6.8 |

(at 25 °C / pH was adjusted by nitric acid or aqueous ammonia)

(Rinse) Common to (1) to (4)

| | | |
|---|---|---|
| Sodium chlorinated isocyanurate | 0.02 g | 0.02 g |
| Deionized water (conductivity: 5 µS/cm or below) | 1000.0 ml | 1000.0 ml |
| pH | 6.5 | 6.5 |

[Processing step ($P_{Blix}$)]

| Processing step | Processing temperature | Processing time | Replenishing rate* | Tank Volume |
|---|---|---|---|---|
| Color developing | 39 °C | 45 sec | 70 ml | 20 liter |
| Bleach-Fixing | 35 °C | 45 sec | 60 ml** | 20 liter |
| Rinse (1) | 35 °C | 20 sec | - | 10 liter |
| Rinse (2) | 35 °C | 20 sec | - | 10 liter |
| Rinse (3) | 35 °C | 20 sec | 360 ml | 10 liter |
| Drying | 80 °C | 60 sec | | |

(* the replenishment rate was the amount per $m^2$ of the light-sensitive material)
(the rinse was conducted in a 3-tank counter-current system of Rinse (3) → (1))
(** in addition to the above 60 ml, 120 ml per $m^2$ of the light-sensitive material was poured from Rinse (1))

To investigate the amount of residual silver after the processing, the multi-layer color photographic printing paper (Sample 001) was exposed uniformly to light, so that the gray density would be 2.2, and then it was processed

in the above processing step ($P_{Blix}$). The amount of residual silver was measured by fluorescent X-ray analysis.

To examine the Blix fading, Sample 001, which was subjected to gradation exposure through a wedge and then was processed in the processing step $P_{Blix}$, was processed again in the below reprocessing step $P_{EDTA}$. By comparing the Dmax values measured with red light (R light) before and after the reprocessing, the extent of the Blix fading was examined.

[Reprocessing step $P_{EDTA}$]

| Processing step | Processing | | Replenishing rate* | Tank Volume |
|---|---|---|---|---|
| | temperature | time | | |
| Bleach-Fixing | 35 °C | 45 sec | 60 ml** | 20 liter |
| Rinse (4) | 35 °C | 20 sec | - | 10 liter |
| Rinse (5) | 35 °C | 20 sec | - | 10 liter |
| Rinse (6) | 35 °C | 20 sec | 360 ml | 10 liter |
| Drying | 35 °C | 60 sec | | |

(* the replenishment rate was the amount per $m^2$ of the light-sensitive material)
(the rinse was conducted in a 3-tank counter-current system of Rinse (6) → (4))
(** in addition to the above 60 ml, 120 ml per $m^2$ of the light-sensitive material was poured from Rinse (4))

The process ($P_{Blix}$) was started by using the above processing solutions and placing the tank solutions in respective processing tanks in the above processing step $P_{Blix}$, and it was continued by adding the replenishers to the respective tanks in accordance with the processed amount. The process was carried out until the accumulated replenishment rate reached three times the tank volume. The results of the processing carried out at this point are shown in Table 2. The reprocess ($P_{EDTA}$) was carried out in the above reprocessing step $P_{EDTA}$ with the chelating agent of the bleach-fix solution changed to ethylenediaminetetraacetic acid. Other components were the same as those in $P_{Blix}$, provided that the reprocess ($P_{EDTA}$) was carried out with the respective solutions being fresh.

Then, to examine the change in performance due to the pH, the same test was carried out with the pH of the mother liquid of the bleach-fix solution changed from 7.0 to 7.5, and with the pH of the replenisher changed from 6.8 to 7.3.

Tests of Nos. 201 to 211 were carried out with the chelating agent used in the bleach-fix solution changed to those shown in Table 2, respectively.

The results are shown in Table 2.

Table 2

| No. | Chelating agent | Amount of residual silver μg/cm² pH 7.0 | pH 7.5 | Blix fading $\Delta D_{max}(R)$ pH 7.0 | pH 7.5 | Remarks |
|---|---|---|---|---|---|---|
| 201 | Comparative compound A | 2.6 | 3.5 | 0.00 | 0.00 | Comparative example |
| 202 | 〃 B | 10.6 | 17.7 | 0.00 | 0.00 | 〃 |
| 203 | 〃 C | 1.8 | 2.5 | 0.19 | 0.13 | 〃 |
| 204 | 〃 D | 1.5 | 3.0 | 0.17 | 0.10 | 〃 |
| 205 | Exemplified compound 1 | 1.0 | 1.1 | 0.00 | 0.00 | This invention |
| 206 | 〃 4 | 0.7 | 0.7 | 0.00 | 0.00 | 〃 |
| 207 | 〃 5 | 1.5 | 1.7 | 0.01 | 0.00 | 〃 |
| 208 | 〃 23 | 1.7 | 1.8 | 0.02 | 0.01 | 〃 |
| 209 | 〃 27 | 1.5 | 1.7 | 0.01 | 0.00 | 〃 |
| 210 | 〃 30 | 1.8 | 1.9 | 0.02 | 0.01 | 〃 |
| 211 | 〃 31 | 1.0 | 1.0 | 0.00 | 0.00 | 〃 |

Comparative compounds A, B, C, and D were the same as those in Example 1.

As is apparent from the results shown in Table 2, the compounds of the present invention are superior to the Comparative compounds in view of the desilvering properties, the Blix fading (fading due to the bleach-fix solution), and the pH dependency of the performance.

Example 3

Sample 301 of a multi-layer color light-sensitive material was prepared in the same manner as in Example 1 of JP-A-5-165176, except that, instead of the cellulose triacetate film base having an undercoat layer used in the multi-layer color light-sensitive material A prepared in Example 1 of JP-A-5-165176, a polyethylene naphthalate base having a thickness of 100 µm was used, and the backing surface of the polyethylene naphthalate base was coated with a striped magnetic recording layer described in Example 1 of JP-A-4-124628. This Sample 301 was subjected to the same test as that for Nos. 101 to 110 of Example 1 of this application, and, similarly to Example 1 of this application, the effects of the present invention were obtained.

Further, Sample 302 of a multi-layer color light-sensitive material was prepared in the same manner as in Example 1 of this application, except that, instead of the base used in the multi-layer color light-sensitive material 101 of Example 1 of this application, the base and the backing layer in Sample No. I-3 in Example 1 of JP-A-4-62543 were used, and the second protective layer was coated with $C_8F_{17}SO_2N(C_3H_7)CH_2COOK$, in an amount of 15 mg/m$^2$. The thus-prepared Sample 302 was worked into a format shown in Fig. 5 of JP-A-4-62543 and was tested in the same manner as for Nos. 101 to 110 of Example 1 of this application, and, similarly to Example 1 of this application, the effects of the present invention were obtained.

Example 4

Sample 601 of a multi-layer color light-sensitive material (color negative film) was prepared by applying the light-sensitive layers described in Kokai-Giho, Kogi No. 94-6023 (published by Hatsumei-Kyokai, 1994), page 96, left column, line 20, to page 114, on a cellulose triacetate film base having an undercoat.

The above Sample 601 was given continuous gradation wedge exposure at a color temperature of 4,800 °K, and then it was subjected to running processing, in the below-shown processing steps using the below-shown processing solutions, with a cine-type automatic processor (until the accumulated replenisher rate of the developer reached three times the tank volume).

The bleach-fix solution was passed through a silver recovering apparatus in an in-line manner, to recover silver. Part of the overflow from the silver recovering apparatus was discharged as waste liquor, and the remainder was regenerated and was reused as a replenisher of the bleach-fix solution. The silver recovering apparatus was a small-sized electrolytic silver recovering apparatus, wherein the anode was made of carbon and the cathode was made of stainless steel, and the current density used was 0.5 A/Bm$^2$. A schematic view of the system for recovering silver is illustrated in Fig. 1 of JP-A-6-175305. That is, the overflow of the bleach-fix solution was connected directly to the silver recovering apparatus, and part of the overflow, in an amount of 100 ml per 1 min, was returned to the original bleach-fix tank, by a pump 1 through a filter. The overflow from the silver recovering apparatus was recovered, in an amount of 300 ml per liter of the overflow, to a regeneration tank. When the recovered amount reached 1 liter, air was bubbled there-through for about 2 hours; then a regenerator was added, and the resulting solution was sent to a replenishing tank of the bleach-fix solution by a pump 2. The remainder of the solution (100 ml) was discharged as waste liquor. The amount of the waste liquor was 220 ml per m$^2$ of Sample 601.

The washing process was carried out using 5-stage multi-chamber water washing tanks arranged side by side in a countercurrent cascade manner. Specifically, use was made of one shown in Fig. 1 of JP-A-5-66540.

The overflow solution of the first washing water $W_1$ was cascaded to the preceding tank of the bleach-fix tank. A reverse osmosis (RO) apparatus RC 30 (manufactured by Fuji Photo Film Co., Ltd.) was placed between the fourth washing $W_4$ and the fifth washing $W_5$. Namely, washing water taken from $W_4$ was passed through the RO apparatus, the condensed solution was returned to $W_4$, and the passed solution was returned to $W_4$. The processing steps are shown below. A schematic diagram of the processor is shown in Fig. 2 of JP-A-6-175305.

| Processing steps | | | | |
|---|---|---|---|---|
| Processing step | Processing | | Replenishing rate*1 | Tank Volume/liter |
| | time | temperature | | |
| Color developing | 1 min 00 sec | 45 °C | 260 ml | 2 |

* the replenishment rate was the amount per m$^2$ of the light-sensitive material

(continued)

| Processing steps | | | | |
|---|---|---|---|---|
| Processing step | Processing | | Replenishing rate*[1] | Tank Volume/liter |
| | time | temperature | | |
| Bleach-Fixing | 1 min 00 sec | 40 °C | 200 ml | 2 |
| Washing (1) | 15 sec | 40 °C | - | 0.5 |
| Washing (2) | 15 sec | 40 °C | - | 0.5 |
| Washing (3) | 15 sec | 40 °C | - | 0.5 |
| Washing (4) | 15 sec | 40 °C | - | 0.5 |
| Washing (5) | 15 sec | 40 °C | 104 ml | 0.5 |
| Stabilizing | 2 sec | Room temperature | 30 ml | Coated |
| Drying | 50 sec | 70 °C | - | - |

* the replenishment rate was the amount per $m^2$ of the light-sensitive material

The crossover time from the color development to the bleach fix, and from the bleach-fix to the first washing, was 3 sec. The average amount carried over per $m^2$ of the light-sensitive material was 65 ml.

The temperature and relative humidity of the atmosphere around the processor were detected by a hygrothermal meter, as shown in JP-A-3-280042, to calculate the evaporated amount, and the evaporation was compensated for each tank. As the water for the compensation for the evaporation, deionized water for the below washing water was used.

In order to make clear the attainable level of the object intended by using the compound of the present invention, the pH of the color developer was forcibly varied. That is, the case wherein the pH was raised by 0.2 (to a pH of 10.25), and the case wherein the pH was decreased by 0.2 (to a pH of 9.85), were tested.

The compositions of the processing solutions are shown below.

| (Color-developer) | Mother Solution (g) | Replenisher (g) |
|---|---|---|
| Diethylenetriaminepentaacetic acid | 4.0 | 4.0 |
| Chelating agent (compound described in Table 3) | 0.01 mol | 0.01 mol |
| Sodium sulfite | 4.0 | 6.0 |
| Potassium carbonate | 40.0 | 40.0 |
| Potassium bromide | 2.0 | - |
| Potassium iodide | 1.3 mg | - |
| 4-Hydroxy-6-methyl-1,3,3a,7-tetrazaindene | 0.14 | - |
| Disodium-N,N-bis(sulfonatoethyl)hydroxylamine | 13.2 | 17.2 |
| 2-Methyl-4-[N-ethyl-N-(β-hydroxyethyl)amino]aniline sulfate | 11.0 | 14.5 |
| Water to make | 1.0 liter | 1.0 liter |
| pH | 10.25 or 9.85 | 10.50 or 10.10 |
| (pH was adjusted by potassium hydroxide and sulfuric acid) | | |

| (Bleach-Fixing solution) | Mother solution (mol) | Replenisher at start (mol) |
|---|---|---|
| 2-{[1-(carboxyethyl)-carboxymethylamino] ethyl}-carboxymethylaminobenzoate iron (III) ammonium monohydrate | 0.08 | 0.13 |
| Ethylenediaminetetraacetic acid iron (III) ammonium dihydrate | 0.10 | 0.17 |
| Aqueous ammonium thiosulfate solution (700 g/liter) | 300 ml | 495 ml |
| Ammonium iodide | 2.0 g | - |
| Ammonium sulfite | 0.10 | 0.17 |
| m-Carboxybenxensulfinic acid | 0.05 | 0.09 |

(continued)

| (Bleach-Fixing solution) | Mother solution (mol) | Replenisher at start (mol) |
|---|---|---|
| Succinic acid | 0.10 | 0.17 |
| Water to make | 1.0 liter | 1.0 liter |
| pH | 6.0 | 5.5 |
| (pH was adjusted by nitric acid and aqueous ammonia) | | |

| (Bleach-Fixing Regeneration agent) Amount added per 1 of recovered solution for regeneration (mol) | |
|---|---|
| 2-{[1-(carboxyethyl)-carboxymethylamino] ethyl}-carboxymethylaminobenzoate iron (III) ammonium monohydrate | 0.05 |
| Ethylenediaminetetraacetic acid iron (III) ammonium dihydrate | 0.07 |
| Aqueous ammonium thiosulfate solution (700 g/liter) | 195 ml |
| Ammonium sulfite | 0.07 |
| m-Carboxybenzenesulfinic acid | 0.04 |
| Succinic acid | 0.07 |

(Washing water) (Both mother solution and replenisher)

Tap water was treated by passage through a mixed bed ion-exchange column filled with H-type strong acidic cation exchange resin (Amberlite IR-120B, trade name, made by Rohm & Haas) and OH-type strong basic anion exchange resin (Amberlite IRA-400, the same as the above) so that the concentrations of Ca ions and Mg ions in water were both made to decrease to 3 mg/liter or below, followed by adding 20 mg/liter of sodium dichlorinated isocyanurate and 150 mg/liter of sodium sulfate. The pH of this water was in the range of 6.5 to 7.5.

| (Stabilizing solution) for coating | (g) |
|---|---|
| Sodium p-toluene sulfinate | 0.03 |
| Polyoxyethylene-p-monononylphenylether (av. polymerization degree: 10) | 0.2 |
| Disodium ethylenediaminetetraacetate | 0.05 |
| 1,2,4-Triazole | 1.3 |
| 1,4-Bis(1,2,4-triazole-1-ylmethyl)piperazine | 0.75 |
| Water to make | 1.0 liter |
| pH | 5.0-8.0 |

After the running processing, the remaining (residual) ratios of the developing agent and the hydroxylamine were found by analysis. Further, it was determined visually if there was a precipitate in the color developer at a pH of 10.25 after the running processing. The results of Nos. 301 to 313, obtained by changing the chelating agent used in the color developer to those shown in Table 3, respectively, are summarized in Table 3.

Table 3

| No. | Chelating agent | Residual amount of developing agent (pH 10.25) | Residual amount of developing agent (pH 9.85) | Residual amount of hydroxylamine (pH 10.25) | Residual amount of hydroxylamine (pH 9.85) | Remarks |
|---|---|---|---|---|---|---|
| 301 | not added | 39 % | 52 % | 34 % | 48 % | Comparative example |
| 302 | Diethylenetriamine-pentaacetic acid | 42 % | 60 % | 38 % | 55 % | 〃 |
| 303 | 1-Hydroxyethylidene-1, 1-diphosphonic acid | 59 % | 71 % | 54 % | 68 % | 〃 |
| 304 | Ethylenediamine-N-carboxy-methyl-N-succinic acid | 43 % | 72 % | 39 % | 70 % | 〃 |
| 305 | Exemplified compound 1 | 79 % | 81 % | 76 % | 78 % | This invention |
| 306 | 〃 3 | 80 % | 83 % | 77 % | 80 % | 〃 |
| 307 | 〃 4 | 81 % | 84 % | 79 % | 81 % | 〃 |
| 308 | Comparative compound 9 | 73 % | 77 % | 70 % | 72 % | 〃 |
| 309 | Exemplified compound 11 | 75 % | 79 % | 70 % | 73 % | 〃 |
| 310 | 〃 19 | 78 % | 82 % | 76 % | 77 % | 〃 |
| 311 | 〃 21 | 70 % | 75 % | 69 % | 70 % | 〃 |
| 312 | 〃 24 | 69 % | 74 % | 69 % | 70 % | 〃 |
| 313 | 〃 29 | 75 % | 78 % | 68 % | 71 % | 〃 |

As is apparent from the results shown in Table 3, when no chelate compound was added, and when a conventional chelating agent was added, the effect of a satisfactory level could not be obtained in some cases, owing to a change in the pH. It can be understood that the addition of the compound of the present invention gives an effect satisfactorily high enough, even if the pH is changed.

Example 5

Sample 702 of the below-shown multi-layer color light-sensitive material was processed, using the processing steps and processing solutions described in Example 1 of this application, with a cine-type automatic processor, and the same evaluation as in Example 1 of this application was effected.

(1) Material of the base

The base used in this Example was prepared by the following method:
PEN: 100 parts by weight of a commercially available poly(ethylene-2,6-naphthalate) polymer, and 2 parts by weight of Tinuvin P.326 (manufactured by Ciba-Geigy), as an ultraviolet absorber, were dried in a usual manner, melted at 300 °C, and extruded from a T-die, and the extrudate was stretched longitudinally 3.3-folds at 140 °C; then it was stretched laterally 3.3-folds at 130 °C, and it was subjected to heat setting for 6 sec at 250 °C. The glass transition temperature of the product was 120 °C.

(2) Application of an undercoat layer

Both surfaces of the above base were subjected to corona discharge treatment, and an undercoat liquid having the below-shown composition was applied, so that an undercoat layer would be provided on the higher-temperature surface during the stretching of the base. The corona discharge treatment was carried out using a solid-state corona treatment apparatus 6KVA model, manufactured by Pillar Inc., and the base, of width 30 cm, was treated at 20 m/min. The subject was treated at 0.375 KV.A.min/$m^2$, which was confirmed from the readings of the electric current and the voltage. The discharge frequency at the treatment was 9.6 KHz, and the gap clearance between the electrode and the dielectric roll was 1.6 mm.

| | |
|---|---|
| Gelatin | 3 g |
| Distilled water | 250 ml |
| Sodium-α-sulfodi-2-ethylhexylsuccinate | 0.05 g |
| Formaldehyde | 0.02 g |

Further, a undercoat layer having the following composition was provided to a TAC support.

| | |
|---|---|
| Gelatin | 0.2 g |
| Salicylic acid | 0.1 g |
| Methanol | 15 ml |
| Acetone | 85 ml |
| Formaldehyde | 0.01 g |

(3) Application of backing layers

The following first to third backing layers were applied to one side of the base having the undercoat made in the above (2).

a) First backing layer

Co-containing needle-like γ-iron oxide fine particles (having an average particle diameter of 0.08 μm and contained as a dispersion in gelatin) 0.2 $g/m^2$

Gelatin 3 $g/m^2$

Compound shown below 0.1 $g/m^2$

$(CH_2=CHSO_2NHCH_2CH_2NH)_2CO$

Compound shown below 0.02 $g/m^2$

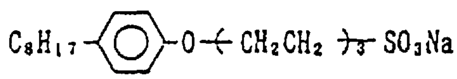

Poly(ethyl acrylate) (having an average diameter of 0.08 μm) 1 $g/m^2$

b) Second backing layer

| | |
|---|---|
| Gelatin | 0.05 $g/m^2$ |
| Electrically conductive material [$SnO_2/Sb_2O_3$ (9 : 1), grain diameter 0.15 μm] | 0.16 $mg/m^2$ |
| Sodium dodecylbenxenesulfonate | 0.05 $g/m^2$ |

c) Third backing layer

Gelatin 0.5 $g/m^2$

Polymethyl methacylate (average grain diameter 1.5 μm) 0.02 $g/m^2$

Cetyl stearate (dispersion with sodium dodecylbenzenesulfonate) 0.01 $g/m^2$

Sodium di(2-ethylhexyl)sulfosuccinate 0.01 $g/m^2$

Compound shown below 0.01 $g/m^2$

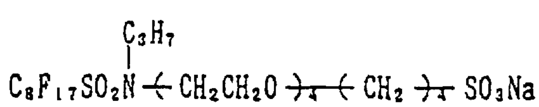

The coercive force of the backing layers thus obtained was 960 Oe.

(4) Heat treatment of the base

After the undercoat layer and the backing layers were applied in the above manner, the base was dried, taken up, and heat-treated at 110 °C for 48 hours.

(5) Preparation of light-sensitive layers

Then, on the side opposite to the backing layers obtained above, was applied light-sensitive layers, described in Hatsumei-Kyokai, Kokai-Giho, Kogi No. 94-6023, page 116, left column, line 17, to page 133, thereby preparing Sample 702 of a multi-layer color light-sensitive material.

The thus-prepared light-sensitive material was cut into a strip having a width of 24 mm and a length of 160 cm. Two perforations of 2 mm square were made at intervals of 5.8 mm, located at the position of 0.7 mm in the width direction and at one side in the lengthwise direction of the light-sensitive material, respectively. Further, sets of such two perforations were made at intervals of 32 mm. The sample was encased in a plastic film cartridge, as illustrated in Fig. 1 to Fig. 7 of US-A-5 296 887.

The above Sample 702 was processed and evaluated in the same manner as in Example 1 of this application. In passing, after it was exposed to light and processed, the Sample 702 was housed again in the original plastic film cartridge.

In the present invention, the light-sensitive material having a magnetic recording layer on the back surface opposite to the emulsion layer gave the similar excellent results as the results of Example 1 of this application.

Example 6

The color paper of Sample 001 of Example 4 of JP-A-5-303186 was processed by using the below-shown processing solutions and processing method.

Parenthetically, in order to make clear the attainable level of the object intended by using the compound of the present invention, the pH of the color developer was forcibly varied. That is, the case wherein the pH was raised by 0.2 (to a pH of 10.25), and the case wherein the pH was decreased by 0.2 (to a pH of 9.85), were tested.

| (Color Developer) | |
|---|---|
| Water | 700 ml |
| Disodium 1,2-dihydroxybenzene-4,6-disulfonate | 4.0 g |
| Triethanolamine | 12.0 g |
| Potassium chloride | 1.5 g |
| Potassium bromide | 0.01 g |
| Potassium carbonate | 27.0 g |
| Fluorescent whitening agent (WHITEX 4, trade name: manufactured by Sumitomo Kagaku Co.) | 1.0 g |
| Sodium sulfite | 0.1 g |
| Disodium-N,N-bis(sulfonatoethyl)hydroxylamine | 10.0 g |
| N-ethyl-N-(β-methanesulfonamidoethyl)-3-methyl-4-amioaniline sulfate | 5.0 g |
| Water to make | 1,000 ml |
| pH (at 25 °C) | 10.25 or 9.85 |

The above color developer was named Sample 401, and those that were prepared by adding the compound of the present invention or Comparative compound thereto in an amount shown in Table 4, were named Samples 402 to 410.

| (Bleach-Fixing solution) | |
|---|---|
| Water | 600 ml |
| Ammonium thiosulfate (700 g/liter) | 100 ml |
| Ammonium sulfite | 40 g |
| Ethylenediaminetetraacetic acid iron(III) ammonium salt | 55 g |

(continued)

| (Bleach-Fixing solution) | |
|---|---|
| Ethylenediaminetetraacetic acid | 5 g |
| Ammonium bromide | 40 g |
| Nitric acid (67 %) | 30 g |
| Water to make | 1,000 ml |
| pH (at 25 °C) (adjusted with acetic acid and aqueous ammonia) | 5.8 |

(Rinse solution)

Deionized water (calcium and magnesium each were 3 ppm or below)

To the above color developers each were added 5 ppm of ferric ions and 150 ppm of calcium ions, and they were allowed to stand at 38 °C for 20 days in beakers, with the opening ratio being 0.10 $cm^{-1}$.

The above color light-sensitive materials were given gradation exposure through a three-color separation filter for sensitometry by using a sensitometer (FWH type, manufactured by Fuji Photo Film Co., Ltd.). The exposure was carried out such that the exposure time was 0.1 sec and the exposure amount was 250 CMS.

After the exposure, they were processed in accordance with the following steps using the above newly prepared color developer (fresh solution) and aged color developer (aged solution).

| Processing method | | | | |
|---|---|---|---|---|
| Processing step | Temperature | Time | Replenishment* rate | Tank volume |
| Color development | 35 °C | 45 sec | 161 ml | 17 liter |
| Bleach-fixing | 35 °C | 45 sec | 215 ml | 17 liter |
| Rinse (1) | 35 °C | 20 sec | - | 10 liter |
| Rinse (2) | 35 °C | 20 sec | - | 10 liter |
| Rinse (3) | 35 °C | 20 sec | 360 ml | 10 liter |
| Drying | 80 °C | 60 sec | | |

(* the replenishment rate was the amount per $m^2$ of the light-sensitive material)
(the rinse was conducted in a 3-tank counter-current system of Rinse (3) to Rinse (1))

The residual amount of the developing agent in the aged solution was determined by high-performance liquid chromatography. It was observed if a precipitation was formed in the aged color developer. The results are summarized in Table 4.

Table 4

| No. | Chelating agent (added amount) | Residual amount of developing agent (%) pH 10.25 | Residual amount of developing agent (%) pH 9.85 | Remarks |
|---|---|---|---|---|
| 401 | not added | 51 | 68 | Comparative example |
| 402 | Sodium hexametaphosphate (1g/liter) | 70 | 82 | 〃 |
| 403 | 1-Hydroxyethylidene-1,1-diphosphonic acid (60%) (1.6 g/liter) | 72 | 85 | 〃 |
| 404 | Ethylenediaminetetraacetic acid | 60 | 71 | 〃 |
| 405 | Ethylenediamine-N-carboxymethyl-N-succinic acid | 56 | 82 | 〃 |
| 406 | Exemplified compound 2 | 84 | 87 | This invention |
| 407 | Exemplified compound 11 | 82 | 85 | 〃 |
| 408 | Exemplified compound 17 | 80 | 82 | 〃 |
| 409 | Exemplified compound 25 | 81 | 85 | 〃 |
| 410 | Exemplified compound 33 | 82 | 86 | 〃 |

As is apparent from the results shown in Table 4, when no chelate compound was added, and when a conventional chelating agent was added, the effect of a satisfactory level could not be obtained sometimes, due to a change in the pH. It can be understood, however, that when the compound of the present invention was added, the residual amount of the developing agent was enough to obtain a satisfactory performances. Further, it can be seen that, with respect to the formation of a precipitate, in comparison with the Comparative Examples, the Examples according to the present invention are improved considerably.

Particularly, out of the conventional compounds, those high in the effect of preventing a precipitate from forming were poor in the preservability of the developing agent, and those that allow the developing agent to be less decomposed were unsatisfactory in the prevention of a precipitate.

In contrast, it can be understood that the compound of the present invention does not allow a precipitate to form, and it provides a stable color developer.

Example 7

The following processing solutions were prepared.

| (Color developer) | (g) |
|---|---|
| Diethylenetriaminepentaacetic acid | 1.0 |
| Chelating agent (described in Table 5) | 0.01 mol |
| Sodium sulfite | 4.0 |
| Potassium carbonate | 30.0 |
| Potassium bromide | 1.4 |
| Potassium iodide | 1.5 mg |
| Hydroxylamine sulfate | 2.4 |
| 4-(N-ethyl-N-β-hydroxyethylamino) -2-methylaniline sulfate | 4.5 |
| Water to make | 1000 ml |
| pH | 10.05 |

5 ppm of ferric ions, in the form of ferric chloride, and 150 ppm of calcium ions, in the form of calcium nitrate, were added to the above color developers, to prepare Samples 501 to 512. 5 liters of each of these samples was filled in a rigid polyvinyl chloride container, of length 10 cm, width 25 cm, and depth 30 cm, and the liquid in the container was continuously circulated by a pump, at 3 liters per min, at the controlled temperature of 38 °C, for 30 days, to carry out an aging test.

Parenthetically, the container was provided with a floating lid, having an area of 200 $cm^2$, for covering the liquid surface, and the liquid surface area exposed to air was 50 $cm^2$.

Multi-layer color negative light-sensitive Sample 101, described in Example 1 of JP-A-4-274236, was cut to a width of 35 mm, and it was given wedge exposure of 5 CMS at a color temperature of 4,800 °K. This was processed in the following processing steps using, as color developers, the freshly prepared solutions (fresh solutions) and aged solutions of Samples 501 to 512.

| [Processing steps] | | |
|---|---|---|
| Processing step | Processing time | Processing temperature |
| Color developing | 3 min 15 sec | 38.0 °C |
| Bleaching | 50 sec | 38.0 °C |
| Fixing | 1 min 40 sec | 38.0 °C |
| Washing (1) | 30 sec | 38.0 °C |
| Washing (2) | 20 sec | 38.0 °C |
| Stabilizing | 20 sec | 38.0 °C |

| (Bleaching solution) | (g) |
|---|---|
| 1,3-Propanediaminetetraacetate Iron(III) ammonium | 0.55 mol |
| Ammonium bromide | 85 |

(continued)

| (Bleaching solution) | (g) |
| --- | --- |
| Ammonium nitrate | 20 |
| Glycolic acid | 55 |
| Water to make | 1000 ml |
| pH | 4.2 |

| (Fixing solution) | (g) |
| --- | --- |
| Ethylenediaminetetraacetic acid ferric ammonium salt | 1.7 |
| Ammonium sulfite | 14.0 |
| Aqueous ammonium thiosulfate solution (700 g/liter) | 260.0 ml |
| Water to make | 1,000 ml |
| pH | 7.0 |

(Washing water)

Tap water was treated by passage through a mixed bed ion-exchange column filled with H-type strong acidic cation exchange resin (Amberlite IR-120B, trade name, made by Rohm & Haas) and OH-type strong basic anion exchange resin (Amberlite IRA-400, the same as the above) so that the concentrations of Ca ions and Mg ions in water were both made to decrease to 3 mg/liter or below, followed by adding 20 mg/liter of sodium dichlorinated isocyanurate and 150 mg/liter of sodium sulfate. The pH of this water was in the range of 6.5 to 7.5.

| (Stabilizing solution) | (g) |
| --- | --- |
| Sodium p-toluenesulfinate | 0.03 |
| Polyoxyethylene-p-monononylphenylether (av. polymerization degree: 10) | 0.2 |
| Disodium ethylenediaminetetraacetate | 0.05 |
| 1,2,4-Triazole | 1.3 |
| 1,4-Bis(1,2,4-triazole-1-ylmethyl)piperazine | 0.75 |
| Water to make | 1000 ml |
| pH | 8.5 |

The B density obtained from the solution that had been subjected to the aging test was measured by an X Light 310-type photographic densitometer, with such an exposure amount that the B density measured with blue light (B light) in the case of the fresh solution would be 2.5, and the difference $\Delta D_B$ from that of the fresh solution was found. The remaining ratios of the developing agent and the hydroxylamine after the aging were found by analysis. Further, with respect to the aged color developer, it was determined visually if a precipitate was formed. The thus obtained results are shown in Table 5.

Table 5

| No. | Chelating agent | $\Delta D_{max}$ | Remaining ratio of developing agent | Remaining ratio of hydroxylamine* | Remarks |
|---|---|---|---|---|---|
| 501 | not added | -0.5 | 60% | 20% | Comparative example |
| 502 | Ethylenediaminetetraacetic acid | -0.4 | 62% | 30% | 〃 |
| 503 | Ethylenediamine-N-carboxymethyl-N-succinic acid | -0.09 | 71% | 62% | 〃 |
| 504 | Exemplified compound 1 | -0.05 | 80% | 73% | This invention |
| 505 | Exemplified compound 2 | -0.05 | 81% | 74% | 〃 |
| 506 | Exemplified compound 3 | -0.04 | 84% | 77% | 〃 |
| 507 | Exemplified compound 4 | -0.04 | 86% | 79% | 〃 |
| 508 | Exemplified compound 27 | -0.07 | 82% | 75% | 〃 |
| 509 | Exemplified compound 28 | -0.07 | 82% | 70% | 〃 |
| 510 | Exemplified compound 31 | -0.04 | 85% | 77% | 〃 |
| 511 | Exemplified compound 33 | -0.05 | 80% | 72% | 〃 |
| 512 | Exemplified compound 34 | -0.04 | 81% | 73% | 〃 |

* Hydroxylamine was oxidized with iodine, and then it was added sulfanyl acid and α-naphthylamine to form (a red color), thereby the remaining ratio of hydroxylamine was determined by spectrophotometory.

As is apparent from the results shown in Table 5, it can be understood that, when the conventional chelating agent was added, the level of securing the prevention of formation of a precipitate and the level of securing the solution stability were unsatisfactory, while with the addition of the compound of the present invention, the effects on securing such properties was remarkably great.

Example 8

The biodegradability of each of ethylenediaminetetraacetic acid (EDTA), ethylenediamine-N-carboxylmethyl-N'-monosuccinic acid (EDMS), and Exemplified Compounds 1 and 4 of the present invention was evaluated in accordance with 301C test of the OECD chemicals test guideline. The results thereof are shown in the table below.

Table 6

| Decomposition ratio in biodegradability test (after 28 days) | |
|---|---|
| Compound | Decomposition ratio |
| EDTA | 0% |
| EDMS | 30% |
| Compound 1 of the present invention | 60% |
| Compound 4 of the present invention | 60% or more |

Thus, it can be understood that the biodegradability of the compounds of the present invention is apparently superior, and the processing solution compositions of the present invention are preferable in view of preservation of the earth's environment.

Having described our invention as related to the present embodiments, it is our intention that the invention not be limited by any of the details of the description, unless otherwise specified, but rather be construed broadly within its scope as set out in the accompanying claims.

## Claims

**1.** A compound represented by the following formula (I):

formula (I)

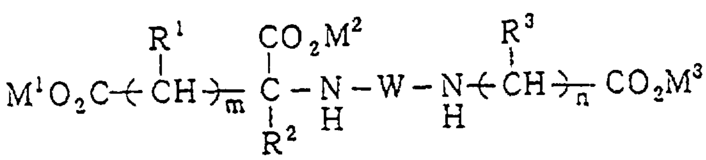

wherein $R^1$ and $R^2$ each represent a hydrogen atom, an alkyl group, an aryl group, a heterocyclic group, a carboxyl group, an amino group, an alkoxy group, a sulfo group, a nitro group, a phosphono group, an acylamino group, a sulfonylamino group, an aryloxy group, a sulfamoyl group, a carbamoyl group, an alkylthio group, an arylthio group, an acyl group, a hydroxamic acid group, or a hydroxyl group; $R^3$ represents a hydrogen atom, an alkyl group substituted with at least one selected from a group consisting of an alkoxy group and an aryl group,

an unsubstituted alkyl group, an aryl group, or a heterocyclic group with the proviso that $R^3$ does not represent an isopropyl group; W represents a divalent linking group having a carbon atom(s); m and n are each an integer of 1 to 3, with the proviso that when n = 1, $R^3$ does not represent a hydrogen atom, when n = 2 or 3, at least one of $nR^3$'s represents an alkyl group, an aryl group or a heterocyclic group, and when m and n are each 2 or more, $mR^1$'s are the same or different and $nR^3$'s are the same or different, and $M^1$, $M^2$, and $M^3$ each represent a hydrogen atom or a cation.

**2.** A compound as defined in claim 1, with the proviso that when n = 2 or 3, one of $nR^3$'s represents an alkyl group, an aryl group or a heterocyclic group and the other $R^3$'s all represent hydrogen atoms and when m is 2 or more, $mR^1$'s are the same or different, and $M^1$, $M^2$, and $M^3$ each represent a hydrogen atom or a cation.

3. The compound as claimed in claim 1 or 2, wherein the compound is represented by the following formula (II):

formula (II)

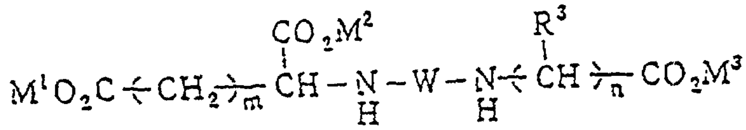

wherein $R^3$, W, m, n, $M^1$, $M^2$, and $M^3$ have the same meanings as those in formula (I).

4. The compound as claimed in claim 1 or 2, wherein the compound is represented by the following formula (III) or (IV):

formula (III)

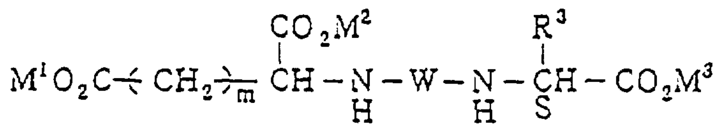

wherein $R^3$, W, m, n, $M^1$, $M^2$, and $M^3$ have the same meanings as those in formula (I), and wherein S indicates that the absolute configuration is the S configuration,

formula (IV)

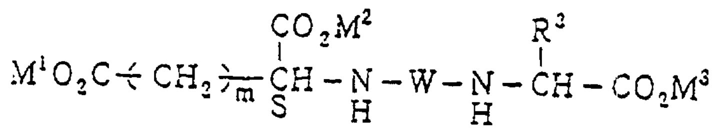

wherein $R^3$, W, m, n, $M^1$, $M^2$, and $M^3$ have the same meanings as those in formula (I), and wherein S indicates that the absolute configuration is the S configuration.

5. A heavy metal chelate compound of the compound represented by formula (I) as defined in any one of the claims 1 to 4.

6. Use of the compound as defined in any one of the claims 1 to 4, as a chelating agent.

7. A photographic additive, comprising the compound represented by formula (I) as defined in any one of the claims 1 to 4 or its heavy metal chelate compound.

8. A method for processing a silver halide photographic light-sensitive material, comprising processing a silver halide photographic light-sensitive material, which has been exposed imagewise, in the presence of at least one compound out of the compounds represented by formula (I) as defined in any one of the claims 1 to 4 or their heavy metal chelate compounds.

9. The method as claimed in claim 8, wherein the at least one compound out of the heavy metal chelate compounds is contained in a processing solution in an amount of 0.005 to 1 mol/liter.

10. Use of the compound represented by formula (I) as defined in any one of the claims 1 to 4 as a component of a

photographic additive to be contained in a silver halide photographic light-sensitive material.

11. Use of the compound represented by formula (I) as defined in any one of the claims 1 to 4 as a component of a photographic additive to be contained in a photographic processing solution.

## Patentansprüche

1. Verbindung, welche durch die folgende Formel (I) dargestellt wird:

Formel (I)

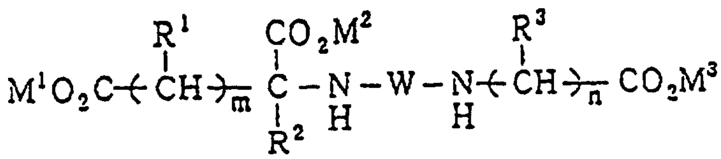

,

worin $R^1$ und $R^2$ jeweils ein Wasserstoffatom, eine Alkylgruppe, eine Arylgruppe, eine heterocyclische Gruppe, eine Carboxylgruppe, eine Aminogruppe, eine Alkoxygruppe, eine Sulfogruppe, eine Nitrogruppe, eine Phosphonogruppe, eine Acylaminogruppe, eine Sulfonylaminogruppe, eine Aryloxygruppe, eine Sulfamoylgruppe, eine Carbamoylgruppe, eine Alkylthoigruppe, eine Arylthiogruppe, eine Acylgruppe, eine Hydroxamsäuregruppe, oder eine Hydroxylgruppe darstellt; $R^3$ eine Wasserstoffgruppe, eine Alkylgruppe, welche durch mindestens eine Gruppe substituiert ist, die aus einer Alkoxygruppe und einer Arylgruppe ausgewählt ist, eine nichtsubstituierte Alkylgruppe, eine Arylgruppe oder eine heterocyclische Gruppe unter der Bedingung darstellt, dass $R^3$ keine Isopropylgruppe darstellt; W eine bivalente Verknüpfungsgruppe darstellt, welche ein Kohlenstoffatom oder Kohlenstoffatome enthält; m und n jeweils eine ganze Zahl zwischen 1 und 3 unter der Bedingung sind, dass, wenn n = 1 ist, $R^3$ kein Wasserstoffatom darstellt, wenn n= 2 oder 3 ist, mindestens eines der n $R^3$ eine Alkylgruppe, eine Arylgruppe oder eine heterocyclische Gruppe darstellt, und, wenn m und n jeweils 2 oder mehr sind, die m $R^1$ gleich oder unterschiedlich sind, und die n $R^3$ gleich oder unterschiedlich sind und $M^1$, $M^2$ und $M^3$ jeweils ein Wasserstoffatom oder ein Kation darstellen.

2. Verbindung, wie sie in Anspruch 1 definiert ist, unter der Bedingung, dass, wenn n = 2 oder 3 ist, eines der n $R^3$ eine Alkylgruppe, eine Arylgruppe oder eine heterocyclische Gruppe darstellt und die anderen $R^3$ alle ein Wasserstoffatom darstellen, und, wenn m 2 oder mehr ist, die m $R^1$ gleich oder unterschiedlich sind, und $M^1$, $M^2$ und $M^3$ je ein Wasserstoffatom oder ein Kation darstellen.

3. Verbindung, wie sie in Anspruch 1 oder 2 beansprucht ist, worin die Verbindung durch die folgende Formel (II) dargestellt ist:

Formel (II)

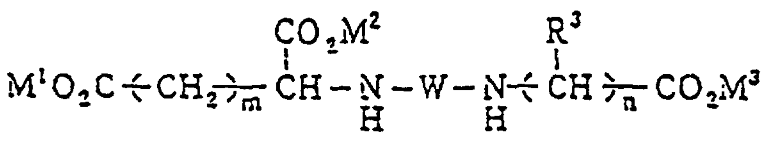

,

worin $R^3$, W, m, n, $M^1$, $M^2$ und $M^3$ dieselben Bedeutungen wie in der Formel (I) haben.

4. Verbindung, wie sie in Anspruch 1 oder 2 beansprucht ist, worin die Verbindung durch die folgenden Formeln (III) und (IV) dargestellt ist:

Formel (III):

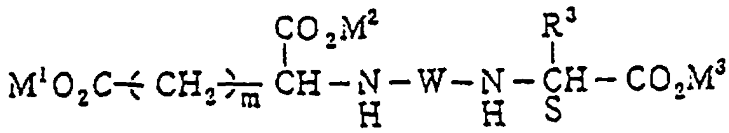

,

worin $R^3$, W, m, n, $M^1$, $M^2$ und $M^3$ dieselben Bedeutungen wie in der Formel (I) haben, und worin S anzeigt, dass die absolute Konfiguration die S-Konfiguration ist,

Formel (IV)

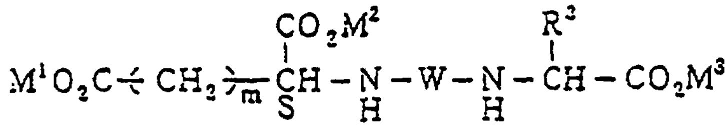

,

worin $R^3$, W, m, n, $M^1$, $M^2$ und $M^3$ dieselben Bedeutungen wie in der Formel (I) haben, und worin S anzeigt, dass die absolute Konfiguration die S-Konfiguration ist.

**5.** Schwermetallchelatverbindung der durch die Formel (I) dargestellten Verbidung, wie sie in irgendeinem der Ansprüche 1 bis 4 definiert ist.

**6.** Verwendung der Verbindung, wie sie in irgendeinem der Ansprüche 1 bis 4 definiert ist, als Chelatbildner.

**7.** Photographischer Zusatz, der die durch die Formel (I) dargestellte Verbindung, wie sie in irgendeinem der Ansprüche 1 bis 4 definiert ist, oder ihre Schwermetallchelatverbindung beinhaltet.

**8.** Verfahren zum Verarbeiten eines photographischen, lichtempfindlichen Silberhalogenidmaterials, wobei das Verfahren beinhaltet, ein photographisches, lichtempfindliches Silberhalogenidmaterial, das bildmäßig belichtet worden ist, in Gegenwart mindestens einer Verbindung aus den durch die Formel (I) dargestellten Verbindungen, wie sie in irgendeinem der Ansprüche 1 bis 4 definiert ist, oder aus ihren Schwermetallchelatverbindungen zu verarbeiten.

**9.** Verfahren, wie es in Anspruch 8 beansprucht ist, worin die mindestens eine Verbindung aus den Schwermetallchelatverbindungen in einer Verarbeitungslösung mit einem Anteil von 0,005 bis 1 Mol/Liter enthalten ist.

**10.** Verwendung der durch die Formel (I) dargestellten Verbindung, wie sie in irgendeinem der Ansprüche 1 bis 4 definiert ist, als Bestandteil eines photographischen Zusatzes, der in einem photographischen, lichtempfindlichen Silberhalogenidmaterial enthalten ist.

**11.** Verwendung der durch die Formel (I) dargestellten Verbindung, wie sie in irgendeinem der Ansprüche 1 bis 4 definiert ist, als Bestandteil eines photographischen Zusatzes, der in einer photographischen Verarbeitungslösung enthalten ist.

## Revendications

**1.** Un composé représenté par la formule (I) suivante :

formule (I)

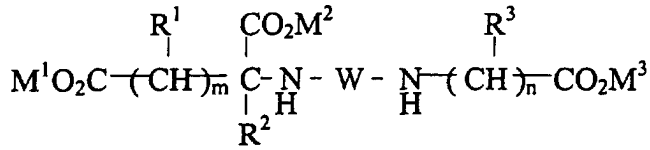

dans laquelle $R^1$ et $R^2$ représentent chacun un atome d'hydrogène, un groupe alkyle, un groupe aryle, un groupe hétérocyclique, un groupe carboxyle, un groupe amino, un groupe alcoxy, un groupe sulfo, un groupe nitro, un groupe phosphono, un groupe acylamino, un groupe sulfonylamino, un groupe aryloxy, un groupe sulfamoyle, un groupe carbamoyle, un groupe alkylthio, un groupe arylthio, un groupe acyle, un groupe acide hydroxamique ou un groupe hydroxyle ; $R^3$ représente un atome d'hydrogène, un groupe alkyle substitué par au moins un substituent séléctionné dans un groupe constitué d'un groupe alkoxy et d'un groupe aryle, un groupe alkyle non substitué, un groupe aryle ou un groupe hétérocyclique, avec la réserve que $R^3$ ne représente pas un groupe isopropyle ; W représente un groupe de liaison divalent ayant un (des) atome(s) de carbone ; m et n sont chacun un nombre entier de 1 à 3 , avec la réserve que lorsque n = 1, $R^3$ ne représente pas un atome d'hydrogène, lorsque n = 2 ou 3, au moins l'un des $nR^3$ représente un groupe alkyle, un groupe aryle ou un groupe hétérocyclique, et lorsque m et n sont chacun 2 ou plus, des $mR^1$ sont identiques ou différents et des $nR^3$ sont identiques ou différents, et $M^1$, $M^2$ et $M^3$ représentent chacun un atome d'hydrogène ou un cation.

2. Un composé tel que défini dans la revendication 1, avec la réserve que lorsque n = 2 ou 3, l'un des $nR^3$ représente un groupe alkyle, un groupe aryle ou un groupe hétérocyclique, et les autres $R^3$ représentent tous des atomes d'hydrogène et lorsque m est 2 ou davantage, des $mR^1$ sont identiques ou différents et $M^1$, $M^2$ et $M^3$ représentent chacun un atome d'hydrogène ou un cation.

3. Le composé tel que revendiqué dans la revendication 1 ou 2, lequel composé est représenté par la formule (II) suivante :

formule (II)

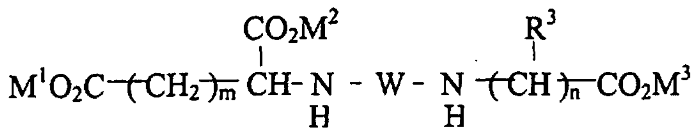

dans laquelle $R^3$, W, m, n, $M^1$, $M^2$, et $M^3$ ont les mêmes significations que celles dans la formule (I).

4. Le composé tel que revendiqué dans la revendication 1 ou la revendication 2, lequel composé est représenté par la formule (III) ou (IV) suivante :

formule (III)

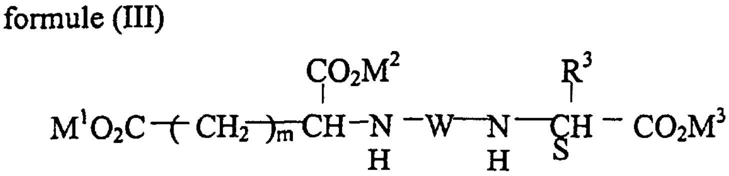

dans laquelle $R^3$, W, m, n, $M^1$, $M^2$, et $M^3$ ont les mêmes significations que celles dans la formule (I), et dans laquelle S indique que la configuration absolue est la configuration S,

formule (IV)

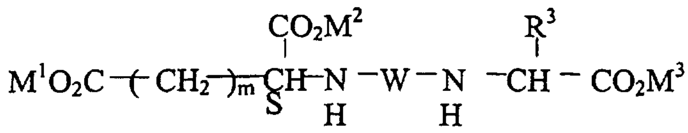

dans laquelle $R^3$, W, m, n, $M^1$, $M^2$, et $M^3$ ont les mêmes significations que celles dans la formule (I), et dans laquelle S indique que la configuration absolue est la configuration S.

**5.** Un composé chélate de métal lourd du composé représenté par la formule (I) tel que défini dans l'une quelconque des revendications 1 à 4.

**6.** Utilisation du composé tel que défini dans l'une quelconque des revendications 1 à 4 en tant qu'agent chélatant.

**7.** Un additif photographique comprenant le composé représenté par la formule (I) tel que défini dans l'une quelconque des revendications 1 à 4 ou son composé chélate de métal lourd.

**8.** Une méthode pour la mise en oeuvre d'un matériau photographique sensible à la lumière à base d'halogénure d'argent, comprenant la mise en oeuvre d'un matériau photographique sensible à la lumière à base d'halogénure d'argent qui a été exposé à une image en présence d'au moins un composé parmi les composés représentés par la formule (I) tel que défini dans l'une quelconque des revendications 1 à 4 ou leurs composés chélates de métal lourd.

**9.** La méthode telle que revendiquée dans la revendication 8, dans laquelle l'au moins un composé parmi les composés chélates de métal lourd est contenu dans une solution de mise en oeuvre en une quantité de 0,005 à 1 mole par litre.

**10.** Utilisation du composé représenté par la formule (I) tel que défini dans l'une quelconque des revendications 1 à 4 en tant que composant d'un additif photographique destiné à être contenu dans un matériau photographique sensible à la lumière à base d'halogénure d'argent.

**11.** Utilisation du composé représenté par la formule (I) tel que défini dans l'une quelconque des revendications 1 à 4 en tant que composant d'un additif photographique destiné à être contenu dans une solution de mise en oeuvre photographique.